# EUROPEAN PATENT APPLICATION

(11) **EP 3 181 153 A1**
(43) Date of publication of application: **21.06.2017**
(21) Application number: 15201170.6
(22) Date of filing: 18.12.2015
(51) Int. Cl.: A61L 15/24, A61L 15/32, A61L 15/58, C08L 1/26, C07K 14/78

(54) **WOUND CARE PRODUCT COMPRISING ECM-FUNCTIONALIZED NANOCELLULOSE**

(71) Applicant: BSN medical GmbH, 20253 Hamburg (DE)
(72) Inventor: Lanfer, Babette, 20149 Hamburg (DE); Arshi, Annahit, 20357 Hamburg (DE); Hemmrich, Karsten, 40589 Düsseldorf (DE); Wilhelms, Tim, 22041 Hamburg (DE); Blumenthal, Nils, 22765 Hamburg (DE)
(74) Representative: Jostarndt Patentanwalts-AG

(57) **Abstract**

The invention relates to a wound care product comprising a scaffold of nanocellulose and least one ECM-mimicking structure. Hereby, the invention preferably relates to a wound dressing with a mixed scaffold of nanocellulose and collagen-like protein fibers. The invention further relates to the use of said wound care product for plastic surgery, tissue engineering and for treatment of acute and chronic wounds. The invention finally relates to a method of producing the ECM-functionalized nanocellulose scaffold.

## Description

### Field of the invention:

The invention relates to a wound care product comprising a scaffold of nanocellulose and at least one ECM-mimicking structure. Hereby, the invention preferably relates to a wound dressing with a scaffold of nanocellulose and embedded collagen-like protein fibers. The invention further relates to the use of said wound care product for plastic surgery, tissue engineering and for treatment of acute and chronic wounds. The invention finally relates to a method of producing the wound care product.

### Background of the invention

Wound healing is a dynamic process involving interactions between cells, extracellular matrix (ECM) and growth factors that reconstitute tissue following injury. The extracellular matrix (ECM) plays an important role in tissue regeneration and is the major component of the dermal skin layer. The composition of ECM includes proteoglycans, hyaluronic acid, collagen, fibronectin and elastin. As well as providing a structural support for cells, some components of the ECM bind to growth factors, creating a reservoir of active molecules that can be rapidly mobilized following injury to stimulate cell proliferation and migration. In many chronic wounds, increased levels of inflammatory cells lead to elevated levels of proteases that appear to degrade the ECM components, growth factors, protein and receptors that are essential for healing.

Recognition of the importance of the ECM in wound healing has led to the development of wound products that aim to stimulate or replace the ECM. These tissue-engineered products comprise a reconstituted or natural collagen matrix that aims to mimic the structural and functional characteristics of native ECM. When placed in the wound bed, the three-dimensional matrix provides a temporary scaffold or support into which cells can migrate and proliferate in an organized manner, leading to tissue regeneration and ultimately wound closure.

As an example, the Integra® Bilayer Matrix wound dressing (Integra LifeSciences) represents a bilayer of bovine tendon collagen and glycosaminoglycan with a polysiloxane (silicone) membrane. However, this wound dressing has the disadvantage of not adhering to the wound site which requires stapling procedures. Moreover, the biological coating does not fully mimic tissue architecture and lacks important biological active components of the ECM such as MMP cleavage sites, growth factors and cytokines.

Hence, there is still a need for an improved wound care product that mimics the ECM and is easy to apply. The objective of the present invention thus is to provide a wound care product which overcomes at least one of the above mentioned disadvantages.

This problem is solved by provision of a wound care product according to claim 1. Specific embodiments are subject matter of further independent claims.

### Summary of the invention:

In a first aspect the present invention provides a wound care product comprising a scaffold of nanocellulose and at least one ECM-mimicking structure which is selected from the group consisting of particulate ECM, collagen protein structure, collagen-like protein structure and peptides comprising a matrix metalloproteinase (MMP) cleavage site or a combination thereof, wherein the at least one ECM-mimicking structure is contained within said nanocellulose scaffold.

The wound care product of the present invention has several advantages over wound care products known in the prior art.

By adding an ECM-mimicking structure it is possible to combine the beneficial properties of the safe and non-noxious nanocellulose layer, which include protection from exterior pollution and bacteria, generation of a humid atmosphere and management of excessive exudate with the reconstructive properties of the ECM.

The nanocellulose layer functions as skin's epidermis, since it protects the wound from infection and controls heat and moisture loss. Furthermore it adheres to the wound site throughout the healing process, so that stapling is not necessary. Once epithelisation is completed and skin has fully regenerated the nanocellulose falls off.

The ECM-mimicking structure as contained within the nanocellulose layer closely resembles native ECM and therefore can act as a scaffold for MMPs to bind to and break down collagen in the product. As a result reduced levels of MMPs are released back into wound as ECM-mimicking structure breaks down, rebalancing protease and growth factor levels in the wound. Furthermore due to further signalling molecules or parts thereof it can induce epithelial cells, fibroblasts and vascular endothelial cells to migrate into the nanocellulose scaffold which then can proliferate.

Due to the reduction in ECM material, the costs for the wound care product can considerably be reduced while the biological ECM activity is retained.

However, the nanocellulose layer of the invention can be provided with additional layers such as, e.g., a covering layer.

By an individual selection of the nanocellulose layer and the ECM mimicking structure, the wound care product can easily be adapted to the specific requirements of the underlying injury. Hence, it offers an enormous flexibility of application.

Since the wound care product of the invention is based on known components, it can be easily produced in a cost efficient manner.

### Detailed description of the invention

The inventive wound care product may be used as such, as a dressing or in combination with a backing known in the art. For example, the wound care product according to the present invention may be temporarily adhered to a backing. This backing may be removed from the wound care product once applied to the wound or after cellular ingrowth has started.

### Nanocellulose

The nanocellulose layer is preferably a layer that consists of nanocellulose.

The nanocellulose as used in the wound care product of the invention is preferably selected from the group consisting of cellulose nanofibers (CNF), nanocrystalline cellulose (NCC) and biocellulose produced by bacteria (BNC).

In a preferred embodiment the nanocellulose layer comprises open pores or at least a porosity which enables cells to enter and grow within the scaffold. Porosity is defined as P=1-ρ∗ (V/M) wherein P is the scaffold porosity, ρ is the density of the polymeric system used, M is the weight and V is the volume of the fabricated scaffolds.

The nanocellulose layer of the invention has a porosity P of more than 50%, more preferably of more than 80%, even more preferably of more than 90% and especially preferably of more than 95%.

In a preferred embodiment the nanocellulose layer is characterized by one or more of the following parameters:
- a thickness of from 0.10 to 0.25 mm,
- a water vapor transmission of more than 300 g/m²*24 h, preferably of more than 500 g/m²*24 h, in particular 1000-3000 g/m²*24 h at 38°C, and/or
- a mass per unit area of 7 to 75 g/m², preferably of 9 to 50 g/m², more preferably of 10 to 40 g/m² and even more preferably of 11 to 22 g/m².

### CNF

In one embodiment of the invention the nanocellulose is provided as cellulose nanofibers (CNF).

Cellulose nanofibers (CNF) are defined as nano-sized fibers consisting of a bundle of stretched cellulose chain molecules with long, flexible and entangled cellulose nanofibers of approximately 1-100 nm size with nano-dimension cross sectional structures. They consist of alternating crystalline and amorphous domains. Nanofibers have extremely large specific surface areas, the properties of which are sometimes significantly different from those of the bulk materials. Cellulose nanofibers (CNFs) form long flexible fiber networks with a fibril diameter similar to or larger than nanowhiskers (CNCs). CNFs can be produced by (2,2,6,6-Tetramethylpiperidin-1-yl)oxyl (TEMPO)-mediated oxidation, multi-pass highpressure homogenization, enzymatic hydrolysis or direct mechanical fibrillation. The morphologies and dimensions of CNFs can vary substantially, depending on the degrees of fibrillation and any pre-treatment involved. CNFs contain amorphous cellulose and are not as highly crystalline as nanowhiskers (CNCs).

Nanowhiskers (CNC) represent highly crystalline and rigid cellulose nanoparticles which are obtained by acid hydrolysis from native fibers and which are shorter (100s to 1000 nm) than cellulose nanofibrils obtained through the homogenization, microfluidization or grinding routes.

The defibrillation of CNF needs intensive mechanical treatment. However, according to degree of processing and raw material, chemical pre-treatments are performed before mechanical fibrillation. The processes for isolating CNF consist of disintegration of cellulose fibers along their long axis. They include simple mechanical methods sometimes in combination with enzymatic or chemical pre-treatments. Appropriate pre-treatments of cellulosic fibers promote the accessibility of hydroxyl groups, increase the inner surface, alter crystallinity, and break cellulose hydrogen bonds and therefore, boost the reactivity of the fibers. Mechanical approaches to diminish cellulosic fibers into nanofibers can be divided into refining and homogenizing, microfluidization, grinding, cryocrushing and high intensity ultrasonication. The resulting aqueous suspensions exhibit gel-like characteristics in water with pseudoplastic and thixotropic properties even at low solid content. Three main technologies, namely homogenization (by means of a Manton-Gaulin homogenizer for example), microfluidization and microgrinding, are widely used for the mechanical treatment. The morphological characteristics of the resulting CNFs depend mainly on those of the original fibers. Generally CNFs obtained from primary cell wall fibers are longer and thinner than those obtained from secondary cell wall fibers.

High pressure homogenization (HPH) process includes passing the cellulose slurry at high pressure into a vessel through very small nozzle. High velocity and pressure as well as impact and shear forces on fluid generate shear rates in the stream and decrease the size of fibers to nanoscale. High pressure homogenization can be considered as an efficient method for refining of cellulosic fibers, because of high its efficiency, simplicity and no need for organic solvents.

Microfluidization is another method similar to high pressure homogenization which can be used to produce CNF. Microfluidization includes the use of an intensifier pump to increase the pressure and interaction chamber in order to defibrillate the fibers using shear and impact forces against colliding streams and the channel walls. Smaller CNF with higher surface area can be obtained by increasing the number of passes through the homogenizer. Also, microfluidization can generate nanofibers with a homogenous size distribution.

Another strategy to break up cellulose into nanosize fibers is grinding, for instance with the help of a static and a rotating grind stone, whereas a pulp slurry passes between these two stones during the process. Fibrillation in a grinder proceeds via breaking down hydrogen bonds and cell wall structures by shear forces, thereby individualizing the pulp to nanoscale fibers.

Cellulose nanofibers having an average fiber length of 250 nm or lower and an average fiber diameter of 2 to 5 nm can be produced, for example, by using pulp obtained by hydrolysis treatment and subsequent kraft cooking as a starting material (known as "dissolved pulp by kraft process" or "DKP"), oxidizing the pulp using an oxidant in the presence of a N-oxyl compound or a compound selected from a group consisting of bromide, iodide and mixtures thereof, and then, defibrating the pulp to form nanofibers. The "pulp obtained by hydrolysis treatment and subsequent kraft cooking (DKP)" means pulp obtainable by kraft cooking of a hydrolyzed plant material, such as wood chip, kenaf, hemp, rice, bagasse or bamboo under general conditions.

The type of a plant material used in the preparation of DKP is not particularly limited. Softwood or hardwood chip which is generally used for pulping, kenaf, hemp, rice, bagasse, bamboo or the like may be used. DKP is characterized in that it has been subjected to hydrolysis as a pre-treatment before kraft cooking. The conditions for the hydrolysis treatment are not particularly limited. For example, the treatment may be performed using an autoclave apparatus or the like to contact water or a liquid-phase or vapour-phase mineral acid with a plant material. After the hydrolysis treatment, a neutralization treatment may be performed by using a mixture of sodium hydroxide and sodium sulfide.

In the patent application CA 2888331 A1 suitable methods for producing CNF are disclosed, the disclosure of said document is disclosed herein by reference.

In the preparation of DKP, the conditions for the kraft cooking performed after the hydrolysis treatment are not particularly limited, but the method used in the preparation of common kraft pulp may be used. For example, in a digester, a cooking liquor containing caustic soda (sodium hydroxide) and sodium sulfide as main components may be added to a plant material to first impregnate the plant material with the cooking liquor and then further cook the plant material.

It is generally accepted that oxidation of cellulose with N-oxyl compounds lead to the formation of carboxyl-groups. Oxidation-derived carboxyl groups are negatively charged, so they are mutually repulsive, and their dispersion in water inhibits aggregation of micro fibrils with each other. Consequently, the fiber bundle is released by microfibril units and form cellulose nanofibers, which are single microfibrils of cellulose.

N-oxyl compounds to be used in oxidizing pulp are compounds that may generate nitroxyl radicals. Among these substances, 2,2,6,6-tetramethyl-1-piperidin-N-oxyradical (referred to hereinafter as "TEMPO") and 4-hydroxy-2,2,6,6-tetramethyl-1-piperidin-N-oxyradical (referred to hereinafter as "4-hydroxy TEMPO") are preferred. Derivatives of these substances can also be used.

The amount of an N-oxyl compound may be a catalytic amount sufficient to oxidize pulp so that the obtained oxidized pulp can be formed into nanofibers.

The oxidized pulp is then defibrated to be transformed to cellulose nanofibers. Defibration may be performed by using a mixing/agitating, emulsifying/dispersing device, such as high-speed shearing mixer or a high pressure homogenizer, alone or by a combination of 2 or more types, as necessary. In the process, the size of oxidized pulp (fiber length and fiber diameter) decreases as the fibers loosen and single microfibrils are formed.

To reduce the energy required for defibrating, the oxidized pulp may be subjected to a suitable cutting (also known as "viscosity-reducing treatment") of the cellulose chains (form short fibers from the cellulose chain) before defibration. Such treatment includes, for example, a treatment that radiates ultraviolet rays on oxidized pulp, a treatment that contacts oxidized pulp with hydrogen peroxide and ozone for oxidative decomposition, a treatment that hydrolyzes oxidized pulp with acid, a treatment that hydrolyzes oxidized pulp with alkali, a treatment with enzymes, such as cellulase, or a combination of these treatments.

### Nanocrystalline cellulose

In one embodiment of the invention the nanocellulose is nanocrystalline cellulose (NCC).

Nanocrystalline cellulose (NCC) is a tightly packed array tightly packed array of microscopic needle-like cellulose crystals. NCC is mainly produced by processing wood pulp. Wood cellulose nanocrystals average 180-200 nm in length with a cross section of 3 - 5 nm and a resulting aspect ratio which varies from 1 to 100, depending on the cellulose source. Nanocrystal dimensions also depend to a certain extent on the hydrolysis conditions used to obtain them.

In a preferred embodiment of the present invention, the nanocrystals of the NCC have an average diameter of less than about 7 nm with substantially all of the nanocrystals have diameters within about 0.5 nm of the average diameter and an aspect ratio of 10 or greater.

The average diameter is preferably less than about 5 nm. In one embodiment, the average diameter is in a range of from about 3 nm to about 7 nm, preferably in a range of from about 3 nm to about 4.9 nm. The aspect ratio is preferably in a range of from about 12 to about 60. Preferably, substantially all of the nanocrystals have diameters within about 0.3 nm of the average diameter.

The NCC as used in the nanocellulose layer preferably has a crystallinity index (CRI) that is 5% or more greater than the CRI of the cellulosic material from which the NCC is made. Advantageously, the CRI may be 7% or more, or even 10% or more than the CRI of the cellulosic material from which the NCC is made. The CRI may be, for example, up to 20% greater, or up to 17% greater than the CRI of the cellulosic material from which the NCC is made. The values of 5% or more, 7% or more or 10% or more may be lower limits of a range in which the upper limit is 20% or 17%. Preferably, the surface carboxylic acid groups are formed by selective oxidation of C6 primary hydroxyl groups of the NCC. Preferably, the degree of oxidation is in a range of from 0.01 to 0.20, more preferably in a range of from 0.02 to 0.2, or in a range from about 0.05 to 0.15, and especially of 0.08.

The most common process for generating nanocrystalline cellulose proceeds via treatment of a cellulose source with a strong mineral acid (such as 64% sulfuric acid), followed by mechanical size reduction. Diverse parent materials can be used, preferably wood pulp. The wood pulp can be prepared from various plants or parts of it such as e.g. cotton, cotton linter, ramie, sisal, tunicate, soft wood and hard wood.

Nanocrystalline cellulose fragments (also known as whiskers, nanowhiskers or nanocrystals) can be generated with variable sizes (widths from 5 to 70 nm and lengths from 100 to several thousand nm). Physical properties of NCC are strongly influenced by source of parent material, the type of acid used in digest (hydrochloric or sulfuric), charge and dimensions.

In an embodiment NCC is produced by controlled acid hydrolysis of cellulose from various sources and preferably from bleached wood pulp. Ground bleached wood pulp is hydrolyzed with 64 % (w/w) sulfuric acid with heating at 45 °C for 25 minutes. The reaction mixture is diluted with water to arrest the hydrolysis and excess acid is removed from the NCC by decantation, centrifugation/washing and dialysis.

In an embodiment, mechanical size reduction processes can be used following the acid digest such as ultrasonic treatment, cryogenic crushing and grinding, and homogenization such as fluidization, which also increase yield. NCC may also be generated from microcrystalline cellulose (MCC) using strong mineral acid hydrolysis followed by separation by differential centrifugation, which results in a narrow size distribution of the NCC.

The optimal conditions can vary considerably, depending on the source of cellulose. In an embodiment the cellulosic material is milled prior to the hydrolysis, usually to pass a to 100 mesh screen. Typically, bleached Kraft pulp from black spruce is milled to pass a 100 mesh screen. An appropriate amount of this pulp, with a water content of about 7% is then added to 60% sulfuric acid kept heated at 60°C. The fluid mixture is stirred for 25 minutes and the hydrolysis is stopped by diluting about tenfold in water. The hydrolysed material is then centrifuged and washed until the pH is ≥ 1 and transferred to a dialysis bag. The removal of free acid is thus pursued until the dialysis water remains close to neutral. The material has then passed partly or wholly in suspension, forming a gel. This gel is treated with a sonifier and the resulting liquid is further polished by a mixed-bed ion exchange resin treatment. The final product spontaneously self-assembles to form a chiral nematic liquid-crystalline phase in a certain concentration range, from about 1% to 20% w/w, preferably 2% to 10% w/w. At lower concentrations the suspension is isotropic and at higher concentrations it is a viscous gel which prevents the formation of the chiral nematic structure. The yield is about 60%. The product is now in its acid form so that sulphur content can be measured by titration. The sulphur content is suitably about 0. 4% to 1%, and is typically found to be of the order of 0.7% by dry weight of the solid. The salt forms of the liquid crystal can be obtained by neutralization with NaOH, KOH, etc. The ionic strength of the preparation can be adjusted by addition of electrolyte, but the suspension will precipitate or form a gel above a certain concentration (a salting-out effect), for example, at about 0.05 M NaCl. If the colloidal suspension is allowed to dry on a support surface, for instance in a petri dish at room temperature, it will form a solid in which the order of the liquid crystal has been preserved. The preserved chiral nematic structure is found to be preferentially planar, i.e., with the long axis of the crystallites preferentially parallel to the substrate on which the film was dried.

In a further embodiment well dispersed colloidal suspensions of NCC are obtained by separating the individual NCC particles by a disruptive treatment such as sonication. The stability of the NCC suspensions derives from anionic sulfate ester groups imparted to the cellulose nanocrystal surfaces during hydrolysis. Hydrochloric acid hydrolysis of cellulose in an analogous manner will also produce NCC; however, the NCC particles are electrostatically neutral (without anionic groups attached) and do not form stable aqueous colloidal suspensions. Post-sulfation by sulfuric acid treatment can be used to impart negatively charged groups to this HCl-produced NCC.

The sulfate ester groups are associated with H⁺ counter ions from the acid hydrolysis, which can be neutralized with a range of inorganic bases (MOH) or organic bases to give salt forms of NCC, (M-NCC) with neutral counter ions other than H⁺, such as alkali metals and in particular Na⁺, K⁺ or Li⁺, or organic phosphonium (R₄P⁺) and organic ammonium ions (R₄N⁺) where each R group which may be the same or different from the other R groups, is an organic chain or group, for example a phenyl group or an alkyl chain of 1 or more, preferably 1 to 4 carbon atoms (e.g., tetraethyl ammonium ion, (C₂H₅)₄N⁺). The acidic NCC is designated H-NCC, while the neutral sodium form of NCC is designated Na-NCC. Thermal treatment, both gentle and harsh, has been used to stabilize NCC films, dried by evaporation, against redispersal in water: heating at 35 °C for 24 h in a vacuum oven is sufficient for solid H-NCC films evaporated at ambient conditions, although heating overnight at 105 °C and at 80 °C for 15 min have also been used to stabilize spin-coated H-NCC films. NCC films containing Na⁺ counter ions have been stabilized at 80 °C for 16 h; as well as at 105 °C for times between 2 and 12 h.

In one embodiment the nanocrystalline cellulose employed in the present invention is derived from cellulose bearing anionic groups, which groups may be associated with cations. In particular, the anionic groups may be sulfate ester groups resulting from hydrolysing of cellulose with sulfuric acid. The cations may, in particular, be alkali metal ions, such as sodium ion or potassium ion.

In a further embodiment, oxidation of biomass from renewable sources to generate NCC with a high degree of carboxylation is conducted in a one-step procedure with ammonium persulfate.

In one embodiment of the invention the NCC is generated by a catalytic reaction process based on breakdown of complex structures into their constituents by reactive oxygen species (ROS) generated from hydrogen peroxide in the presence of a transition metal catalyst, at an acidic pH.

In another embodiment of the present invention, the NCC is produced by a method comprising the following steps: Providing a cellulosic material; contacting the cellulosic material with an inorganic persulfate at an elevated temperature to produce cellulose nanocrystals; and, recovering the cellulose nanocrystals. The inorganic persulfate preferably comprises ammonium persulfate ((NH₄)₂S₂0₈), sodium persulfate (Na₂S₂0₈), potassium persulfate (K₂S₂0₈) or a mixture thereof. More preferably, the inorganic persulfate comprises ammonium persulfate, (NH₄)₂S₂0₈. In this embodiment, any suitable source of cellulosic materials may be used, for example, vegetative or non-vegetative biomasses. Non-vegetative biomasses include cellulosic materials that have undergone considerable pre-treatments, for example, cellulose from papers and microcrystalline cellulose (MCC). In the described embodiment NCC may be produced in one step from vegetative biomass. Thus, the cellulosic material preferably comprises vegetative biomass, more preferably raw vegetative biomass. Any suitable vegetative biomass may be used, for example, one or more of hemp material (e.g. raw hemp, pectate-lyase treated hemp), flax material (e.g. raw flax, pectate-lyase treated flax), triticale material, wood sources (e.g. wood pulp, cardboard) and agricultural residues. More preferably, the cellulosic material comprises one or more of hemp or flax material. The process is conducted at an elevated temperature. Preferably, the elevated temperature is in a range of from about 45°C to about 80°C, for example about 60°C. The persulfate is preferably provided in an aqueous solution. The aqueous solution preferably has a concentration of persulfate in a range of from about 0.5 M to about 2.0 M, more preferably in a range of from about 0.5 M to about 1.0 M, for example about 1.0 M. Preferably, the persulfate is stirred with the cellulosic material. Preferably, contacting the cellulosic material with persulfate is performed for a period of time in a range of from about 5 hours to about 24 hours, for example about 16 hours.

In a preferred embodiment of the invention the NCC is provided as a film. In a preferred embodiment this film is produced from an aqueous suspension of NCC whereby the process is more preferably performed as follows: The aqueous NCC suspension is typically spaced from a source of heat whereby a heat transfer zone is disposed between the suspension and the source of heat. This heat transfer zone may typically be air. Suitably, the suspension itself is in the form of a thin liquid layer. Applying heat in this way from the heat source through the heat transfer zone results in a relatively uniform transfer of heat from the source to the suspension. With the evaporation of water from the suspension, there is thereby formed a film.

The heat transfer rate of the heating zone is suitably one sufficient to permit evaporation of water from the suspension with formation of the required solid film. In particular, the evaporation time is suitably and sufficient to permit the self-assembly of the NCC particles into a chiral nematic organization; a typical suitable range of time for the evaporation is 4 to 6 hours.

The solid film produced may be a solid film of the NCC, or may be a plasticized film of NCC achieved by including a plasticizer such as polyvinyl alcohol in the suspension.

In another embodiment of the invention, material such as plasticizers, polymer resins, or reinforcing agents (woven or non-woven fibres of glass, carbon, wood, etc.) can be added to the dispersion. For instance, a plasticizer such as glycerol will make the films more pliable; a water soluble resin, such as a melamine resin will have the same effect, as well as a strengthening effect. Such a resin may or may not be cross-linked to the crystallites.

### BNC

In a preferred embodiment of the invention the nanocellulose is a biocellulose produced by a microorganism, which preferably is a bacterium of the genus *Acetobacter.*

In one embodiment of the invention two or more strains of *Acetobacter* might be used in combination to manufacture matrices of varying nanocellulose strands, which may be brought together to form nanocellulose of varying layers, levels and controlled three dimensional structures.

In one embodiment, the present invention comprises at least one multi-ribbon biocellulose produced by, e.g., *Acetobacter xylinum* (which is synonymous to *Komagataeibacter xylinus*).

A further embodiment of the present invention comprises at least one multi-ribbon biocellulose which is produced by *Acetobacter xylinum* strain NQ-5 under conditions in which the biocellulose ribbons are deposited at the liquid gas interface and/or under shaking conditions.

Certain strains are known in prior art that yield cellulose with reversal of direction of cellulose ribbon extrusion. The reversal of direction of cellulose ribbon extrusion results from the cellulose-producing microorganism shuttling, at least periodically, first in one direction and then in the other direction along a length of an earlier-deposited cellulose ribbon to add another cellulose ribbon thereto and produce a cellulose ribbon-bundle having a width of at least two cellulose ribbons. The cellulose producing microorganism of the present invention may be at least one of the genuses *Sphaerotilus, Pseudomonas spp., Alcaligenes spp., Salmonella spp., Agrobacterium spp., Rhizobium spp.,* any cyanobacterium that produces cellulose or is genetically modified to produce cellulose such as *Nostoc, Scytonema, Synechococcus, Agmenellum* or *Anabaena.* Preferably, the cellulose producing microorganism of the present invention may be at least one of the genus Acetobacter and more preferably of the species *Acetobacter xylinum* or *Acetobacter pasteurianus.* Among preferred *Acetobacter xylinum* strains are strain NQ5, H1A; H1B; H1C; H2A; H2B; H5C; H5D; H6A; H6C; H7B; H8C; H8G; H9D; H10C; H14B; H15A; and H15B. *Acetobacter xylinum* strain NQ5, has particularly useful characteristics and has deposit No. ATCC 53582 with the American Type Culture Collection, Rockville, MD.

One example of microorganisms that produce a nanocellulose include members of the genus Acetobacter or bacteria, microorganisms or organisms or tissues that have been transformed (permanently or transiently) with one or more genes capable or required for manufacturing cellulose and strains or sub-strains related to or derived therefrom. The nanocellulose may be a cellulose derivative, such as carboxymethyl-nanocellulose, methyl-nanocellulose, hydroxyethyl-nanocellulose, hydroxypropyl-nanocellulose and hydroxypropylmethyl-nanocellulose or mixtures or combinations thereof. The fibrous organic substrate may be a microfibrillar cellulose that is wet, partially wet, dry, anhydrous, hydrated, coated or uncoated at a submicron thickness.

In a preferred embodiment of the invention the cellulose is recombinantly expressed in the microorganism. Hereto, a suitable microorganism is transformed with a vector comprising at least one gene derived from a bacterial cellulose synthase operon, and the transformed microorganism is then cultured under conditions suitable for production of cellulose. In an embodiment, the chromosomal cellulose synthase promoter can be replaced with a heterologous promoter to overexpress an intrinsic cellulose synthase operon at the chromosomal level.

A wide variety of cellulose operons and promoter systems may be used in order to facilitate a nanocellulose production in a genetically modified organism, e.g., the cellulose operon acsABCD from NQ5 under the control of an PrbcL promoter from *Synechococcus leopoliensis,* a portion of the cellulose operon sufficient to express bacterial cellulose that includes the acsAB genes from the cellulose synthase operon of *Acetobacter sp.*

In a preferred embodiment, the portion of the cellulose operon sufficient to express biocellulose may include the acsAB genes from the cellulose synthase operon of the gram negative bacterium *Acetobacter xylinum* or from the *Acetobacter* multiribbon strain NQ 5. It has been found that it is possible to manufacture cellulose with a lower crystallinity than wild-type bacterial cellulose, amorphous cellulose. In one embodiment of the present invention, the cellulose genes are from mosses (including *Physcomitrella*), algae, ferns, vascular plants, tunicates, and combinations thereof. In yet another non-exclusive embodiment, the cellulose genes are selected from gymnosperms, angiosperms, cotton, switchgrass and combinations thereof. The skilled artisan will recognize that it is possible to combine portions of the operons of bacterial, algal, with fungal and plant cellulose genes to maximize production and/or change the characteristics of the cellulose.

In an embodiment the bacterial nanocellulose is produced by a cyanobacterium, which is transformed with a vector for expression of a portion of the cellulose operon sufficient to express bacterial cellulose operon that includes a microbial cellulose operon, e.g., the acsAB gene operon, under the control of a promoter that expresses the genes in cyanobacteria. The skilled artisan will recognize that the vector may combine portions of the operons of bacterial, algal, fungal and plant cellulose operons to maximize production and/or change the characteristics of the cellulose and may be a transfer and/or expression vector.

As used herein, the term *Acetobacter* refers to a genus of microorganisms, and in particular, to the members of that genus that produce cellulose. Although a number of microorganisms fitting this description are known, their taxonomic classification has been subject to debate. Thus, any cellulose producing strain of *Acetobacter* whether classified as *Acetobacter aceti* subsp. *xylinum, Acetobacter pasteurianus* or otherwise, that has the characteristics of stability under agitated culture conditions as further explained below, is considered to be within the scope of the invention.

The cellulose ribbon-bundles produced by the cellulose-producing microorganism of the present invention may be characterized as comprising antiparallel cellulose ribbons having beta-1,4 linkages.

In an embodiment, microorganisms producing nanocellulose do so by proceeding in a first direction alternating with cellulose ribbons having beta-1, 4 linkages proceeding in an opposite direction. This alternating structure results from reversals in the direction of cellulose ribbon extrusion from a microorganism traveling along a previously deposited cellulose ribbon and depositing a new cellulose ribbon which becomes hydrogen-bonded to the adjacent earlier deposited cellulose ribbon. The cellulose of the present invention is preferably produced by a cellulose-producing microorganism aerobically cultivated in an aqueous nutrient medium having a pH between about 3 and about 7 and at a temperature between about 20 °C and about 40 °C.

The nanocellulose is preferably produced in a static cultivation environment.

The typical Hestin Schramm medium is only useful if the peptone is from an animal free source. Otherwise the TSE/BSE risk has to be evaluated with respect to the use as a medical device.

Depending to the global region several cost efficient media can be used:

### Asia: Nata de Coco Formulation:

### Materials:

- 1 kg grated coconut
- 600 g refined sugar
- 325 c glacial acetic acid
- 1/2 L Coconut water
- 12 L Ordinary water
- 2 L Nata starter

### Preparation method:

1. Grate 2-3 coconut heads.
2. Add 1 L of water and squeeze the coconut meat.
3. Filter to remove the residue.
4. Add 1 L of water to the coconut meat and extract the milk again.
5. Combine the first and the second extracts and add to 26 L of water
6. Add the sugar, acetic acid and the mother liquor.
7. Mix well to dissolve the sugar and other ingredients.
8. Fill into containers of about 2-3 cm.. thick.
9. Cover with paper and tighten with the rubber band.
10. Place in a room where temperature is 28-32°C.
11. Leave the mixture undisturbed for 8-10 days.
12. Harvest nata when it is about 1-1.5 cm thick. The yield of one formulation is 20-25 kilos raw nata.

Temperature optimum: 30°C +-2°C
pH optimum: 1,5-4

### Thailand medium:

Use of coconut water instead of coconut milk and addition of magnesium sulfate and ammonium nitrate.

### Regular Hestrin Schramm medium:

+ Trace elements as disclosed in paragraph [0026] of EP 1356831 A1 For a medical device it is essential to make after the harvesting a depyrogenisation step. After the cellulose film has been produced, the cells have to be removed from the cellulose pellicle for purification. Fontana et al. (1990, Appl. Biochem. Biotech, 24: 253-264) have described the cells as being apyrogenic, however, a further cell removal process is often required as disclosed in in EP 1356 831 A1 in order to pass the standard pyrogenicity test and qualify the microbial cellulose wound dressing as nonpyrogenic.

A soft depyrogenisation treatment is also described in a Publication from Klemm et al. (Adv. Polym. Sci 2006, 205: 49-96). Only a low sodium hydroxide concentration is added (0,1%) plus a tenside like SDS. The "depyrogenisation rinsing treatment as taught by Klemm et al is equal to a standard treatment with approx. 3% sodium hydroxide.

To reduce the cost for the medium it is also possible to use corn steep liquor instead of peptone in the Hestrin Schramm medium. Furthermore the glucose in the Hestrin Schramm medium can be supplemented with sucrose. (see EP 1 356 831 A1 paragraphs 26 to28 or Jung et al., 2010, App. Biochem. Biotechnology, 162: 486-497).

In an embodiment the cellulose-producing microorganism is capable of repeated reversals of direction of cellulose ribbon extrusion. Such directional reversals of extrusion result in said cellulose-producing microorganism shuttling, at least periodically, first in one direction and then in the other direction along a length of one or more earlier extruded cellulose ribbons to add another cellulose ribbon thereto. This process produces a cellulose ribbon-bundle being stronger than that produced by non-shuttling microorganisms. These ribbon-bundles have a width of at least two, and preferably three or more, cellulose ribbons. The cellulose thus produced may be collected for use or further processing, if desired. The microorganisms and conditions described above may be used to form this product cellulose comprising cellulose ribbon-bundles with cellulose ribbons of alternating antiparallel orientation, also described above. These antiparallel ribbon alignments result in glucan chain interrelationships reminiscent of cellulose II structure. This cellulose product of a reversal-type cellulose producing microorganism may thus be a polymorph of cellulose I and cellulose II. The antiparallel ribbon alignment should result in one additional inter-ribbon hydrogen bond for each pair of interacting glycosidic units. This additional hydrogen bond, along with the greater numbers of ribbons in a bundle, is consistent with an apparent greater physical strength observed.

In an embodiment the manufacturing of the bacterial nanocellulose includes the step of growing at least one layer of a bacterial nanocellulose substrate in a vessel that serves as a template for the shape of the dressing. In an embodiment, this template may be preformed based on, e.g., a scanned image or other measurements of the wound location. One or more wound dressings may be prefabricated to serve as replacements for the respective prior dressing during the recovery of the wound site, e.g. in case of grafting operations in which the wound may take, days, weeks or even months to heal and/or for patients that have slow wound healing processes.

In an embodiment of the invention, the wound dressing may be grown, assembled or synthesized on a substrate that provides a template for the shape of the dressing. In a preferred embodiment, the dressing comprises reservoirs for the external addition of at least one active agent. In a preferred embodiment, the substrate that provides a template for the shape of the dressing also provides elements for the formation of at least one reservoir for the growth of cells or the deposition of at least on biologically active agent in the nanocellulose.

Using the present invention, the nanocellulose can also be designed to provide diversity in structure and functionality because before, during or even after synthesis it is possible to laminate or sandwiched the strings, sutures, sheets and/or membranes either from the same organism, or from different organisms. The manufacturing process can be applied after synthesis; however, the actual use of two or more nanocellulose manufacturing strains may be used at the same time to generate hybrid membranes of cellulose with physical features that are different from either grown alone. Methods of cellulose molding during synthesis are taught, generally as published in European Application No. EP 0 186 495, which teaches a process for microbial cellulose production; relevant techniques, materials and methods incorporated herein by reference. A liquid culture of cellulose-producing microorganism and a structure are taught. The structures may be made in the presence of an oxygen-containing gas to create, if required, a gas permeable material. Otherwise, the structure is generally not gas permeable. Agents may be added during synthesis to alter the configuration of microbial cellulose. These same techniques may be applied to the nanocellulose of the present invention.

In one embodiment the bacterial nanocellulose of the present invention is synthesized by bacteria, preferably the bacteria *Acetobacter xylinum,* and was recovered from inoculation flasks and propagated via continued inoculation and incubation for linear growth in subsequent flasks and carboys of optimized media to attain the desired volume of microbially derived cellulose. The media is comprised of nutrients such as sucrose, ammonium sulfate, sodium phosphate, magnesium sulfate, citric acid, acetic acid and trace elements resulting in a growth media having a pH of about 4.0 to about 4.4. The sterilized media is inoculated from propagation cultures of *A. xylinum* and filled into bioreactor trays at the appropriate volume to yield a final cellulose to water ratio of about 90% to 95% water to about 5% to 10% cellulose. The bioreactor trays are sealed and incubated in a controlled environment at 30 °C ± 2 °C until growth of a pellicle of bacterial nanocellulose is complete. The pellicles are removed from the bioreactor trays and are chemically treated to remove bacterial by-products and residual media. A caustic solution, preferably sodium hydroxide at a preferable concentration of about 0.1 M to 4 M, is used to remove viable organisms and pyrogens (endotoxins) produced by bacteria from the pellicle. The treated pellicles are then rinsed with filtered water to reduce microbial contamination (bioburden).

Further processing of the present invention continues with the use of a water-miscible organic solvent selected from the group consisting of methanol, ethanol, propanol, isopropanol, acetone and mixtures thereof to dehydrate the cellulose. Without being bound to any one theory, it is believed that soaking the compressed films in a water-miscible organic solvent cross-links the cellulose fibers, thereby yielding a product having increased tensile strength, reduced elongation (stretch) and increased suture retention when used as an implantable medical device for various surgical procedures.

In an embodiment the present invention undergoes depyrogenation by using a heated caustic solution (0.1 M to 4 M sodium hydroxide) known to destroy endotoxins that may be present due to bacteria or cross-contamination from materials exposed to pyrogens. Preferably, the material is then gamma irradiated at doses sufficient to destroy microorganism contamination by pre-determined sterility assurance levels based on bioburden levels (the amount of microorganisms typically present on the non-sterile material.) Samples were gamma irradiated at a dose of about 35 kGy. It can be concluded that the material can be depyrogenated with a strong alkaline sodium hydroxide solution at an elevated temperature and that it can withstand gamma sterilization without any significant affect to mechanical properties.

The cellulose pellicle is subjected to a series of chemical wash steps to convert the raw cellulose film into a medical grade and non-pyrogenic wound dressing material. Typical processing uses hydroxide solutions at concentrations of 1 to 20% by weight. Preferably, sodium hydroxide is used at a concentration of not less than 3% and most preferably about 3% to about 5% in order to acetylate and eventually dissolve the cells. In addition, according to the invention the cellulose films can be treated by hydrogen peroxide washing capable of bleaching and sterilizing the pyrogen free films. Concentrations of about 0.05% to about 10% peroxide by weight are useful to effect whitening of the films. Preferably the amount of peroxide used in about 0.1 % to about 0.5%.

The cellulose fibrils produced by these microorganisms, although chemical resembling, in many aspects, cellulose produced from wood pulp, are different in a number of respects. Chiefly among the differences is the cross sectional width of these fibrils. The cellulose fibrils produced by *Acetobacter* are usually two orders of magnitude narrower than the cellulose fibers typically produced by pulping birch or pin wood. The small cross sectional size of these *Acetobacter-*produced fibrils, together with the concomitantly greater surface area than conventional wood-pulp cellulose and the inherent hydrophilicity of cellulose, leads to a cellulose product having unusually great capacity for absorbing aqueous solutions.

A use of *Acetobacter xylinum* to coat synthetic fibers with microbial cellulose is disclosed in U.S. 4,378,431 A1, which is incorporated by reference herein.

In a preferred embodiment, a bioresorbable bacterial nanocellulose is used. Variable degrees of oxidation affect the rate of resorption. For example, the microbial cellulose may be subjected to oxidation at various degrees to render it bioresorbable. This resorption can be tailored to individual needs so that voids are not created by the material degrading too quickly. Preferably, the degree of oxidation is between 10 and 30 %.

The degree of oxidation of the bacterial nanocellulose is achieved by varying a factor such as concentration and volume of oxidizing agent, a ratio of oxidizing agent to microbial cellulose, reaction temperature and duration, and combination thereof. For example, the molarity of oxidizing agent may be in the range of 0.005 M to 0.5 M, and the temperature may be from about 5 to 50 °C. Preferably, the microbial cellulose may be oxidized for at least 30 minutes, or 1, 6, 12, or 24 hours, or longer. In a preferred embodiment, the microbial cellulose may be oxidized with a solution containing sodium periodate such as sodium meta periodate and optionally, a supporting electrolyte such as NaCl. In a specific embodiment, the microbial cellulose paste may be oxidized by incubating the paste in 80 mM NAlO₄ for 18 to 20 hrs at 30 °C.

The final level of oxidation for the microbial cellulose can be tailored to the specific product application, but preferably, the level of oxidation is sufficient to render the microbial cellulose bioresorbable at a desired rate of degradation including rates ranging from as little as one day to over one year. In other words, the oxidized microbial cellulose can be prepared so as to be resorbable in about one, two, three, four, or five days, about one, two, or three weeks, about one, two, three, four, five, six, seven, eight, nine, ten, or eleven months, or about one or two years.

### Further bioactive molecules

A preferred nanocellulose layer for the wound care product of the invention further contains bioactive molecules which might be already present in the nanocellulose layer or alternatively added to the nanocellulose layer after preparation. For example, the nanocellulose layer may contain at least one compound from a group including fibroblast growth factor-2 (basic FGF), transforming growth factor-beta (TGF-beta) platelet derived growth factor (PDGF), cartilage derived growth factor (CDGF), epidermal growth factor (EGF), transforming growth factor alpha (TGF-alpha), thrombospondin, osteopontin, angiotensin converting enzyme (ACE), bone morphogenetic protein (BMP), NGF (nerve growth factor), tumor necrosis factor alpha (TNF-α), acidic fibroblast growth factor (aFGF), hepatocyte growth factor (HGF), insulin-like growth factors 1 and 2 (IGF-1 and IGF-2), stromal derived factor 1 alpha (SDF-1 alpha), ciliary neurotrophic factor (CNTF), neurotrophin-3, neurotrophin-4, neurotrophin-5, pleiotrophin protein (neurite growth-promoting factor 1), midkine protein (neurite growth-promoting factor 2), brain-derived neurotrophic factor (BDNF), tumor angiogenesis factor (TAF), corticotrophin releasing factor (CRF), interleukin-8 (IL-8), granulocyte-macrophage colony stimulating factor (GM-CSF), and/or vascular endothelial growth factor (VEGF). Further preferred is that the ECM material used in the present invention contains additional bioactive components including, for example, one or more of collagens, glycosaminoglycans, glycoproteins and proteoglycans. The ECM material may further include the basement membrane or parts thereof, which are typically made up mostly of type IV collagen, laminins and proteoglycans.

In various embodiments the bioactive molecules, being preferably growth factor(s) are released slowly and sustained over at least one day, or over at least 3 days or over at least 5 days, or over at least one week, or over at least two weeks, or over at least 3 weeks, or over at least one month, or aver at least two months, or aver at least three months, or over at least six months. Typically the growth factor(s) are released directly to the target tissue in the body.

In an embodiment of the invention, the nanocellulose layer comprises silver nanoparticles.

### ECM-mimicking structure

According to the invention the ECM-mimicking structure is one or more of the following: particulate ECM, collagen protein structure, collagen-like protein structure and peptide comprising a matrix metalloproteinase (MMP) cleavage site.

The particulate ECM as directly deriving from natural ECM comprises the relevant components for providing an ECM-like biological function. The further components, namely the collagen protein structure, the collagen-like protein structure or the peptide comprising a matrix metalloproteinase (MMP) cleavage are modified to exhibit ECM-like properties.

### Particulate ECM

In one embodiment of the invention the hydrogel layer comprises particulate ECM as an ECM-mimicking structure.

A hydrogel nanocellulose layer has a very high water content. After harvesting the water content is approx. 200g water: 1g cellulose. For a medical device it is useful to reduce the water content in the nanocellulose sheet with e.g. pressure. A respective method is disclosed in the patent application EP 1 356 831 A1. The Suprasorb X product (Company Lohmann and Rauscher, Neuwied, Germany) has a water cellulose ration of approx. 96g water:4g cellulose. The wet sheet 21*14cm has a weight of approx. 12g per dm² and a dry weight of approx. 0.45g.

In a preferred embodiment the ECM content should be approximately between 2 wt% and 20 wt% as measured for the dry weight of nanocellulose. In a more preferred embodiment the ECM content is in a range between 5 wt% and 15 wt% an even more preferably is approx. 10 wt% with regard to the dry nanocellulose.

The extracellular matrix (ECM), as understood in the context of the present application, is the non-cellular portion or material of animal or human tissues that surrounds cells. The ECM material may be obtained from any kind of tissue by the use of physical, enzymatic, and/or chemical methods well known in the art. In principal, the major components of ECM comprise fibrous elements (particularly collagen, chitosan, elastin or reticulin), link proteins (for example fibronectin or laminin) and space-filling molecules (for example glycosaminoglycans). Moreover, ECM typically includes growth factors, cytokines, and other biological molecules that are known to attract cells and to promote cellular proliferation.

For preparing the particulate ECM the skilled person can draw on a broad spectrum of potential ECM sources. In one embodiment the ECM can be isolated from *in vitro* cultivated cells. Preferably cells include cultured chondrocytes, fibroblasts, keratinocytes or epithelial cells.

The ECM material for preparing the particulate ECM used in the present invention may be obtained from tissue harvested from any animal, but in particular from animals raised for meat production, including but not limited to, pigs, cattle and sheep. Using these animals as sources for the ECM material reduces the costs of the material. Other warm-blooded vertebrates may also hold as a source of tissue. Moreover, the ECM material may also be obtained from tissue of human corpses. The ECM material can be obtained from any organ, but is preferably obtained from intestinal tissue, bladders, liver, spleen, stomach, lymph nodes or skin. Preferably, the ECM material used in the present invention is derived from human cadaver skin, porcine urinary bladder submucosa (UBS), porcine urinary bladder matrix (UBM), or porcine small intestinal submucosa (SIS). When human tissue is used as the source of the ECM material, the risk of an immunogenic response or immune rejection can be reduced. However, using ECM material of animal origin may reduce the risk of a transfer of diseases. In a preferred embodiment of the present invention, the ECM material is of animal origin. Particularly preferred is an ECM material derived from warm-blooded mammals, due to their similarity to humans. Preferably, the ECM material used in the present invention is of porcine origin. Most preferred is an ECM material derived from Urinary Bladder Matrix (UBM) or small intestine submucosa (SIS) of porcine origin.

In a preferred embodiment of the invention the particulate ECM is preferably a decellularized ECM. Hereby, the isolated tissue is treated with a series of oxidizing agents that remove the cellular and nuclear material from the isolated tissue while substantially retaining the biological and mechanical properties, as well as the biochemical composition of the resulting ECM.

Decellularizing agents that can be used in the methods of the invention are those agents that are effective in removing cellular and nuclear material from the isolated tissue without substantially compromising the biocompatible, biological and mechanical properties or the biochemical composition of the ECM. Examples of decellularizing agents that can be used for decellularization of the tissue include, but are not limited to, oxidizing agents (e.g., hydrogen peroxide, peroxy acids), ascorbic acid (vitamin C), chelating agents (e.g., EDTA, EGTA), methionine, cysteine, maleic acid, and polymers that bind to DNA (e.g., Poly-L-lysine, polyethylimine (PEI) and polyamidoamine (PAMAM)).

In a further preferred embodiment of the invention, the particulate ECM has a mean diameter of approx. 150 µm. This is determined by a Mastersizer 200 from Malvern Instrument for volume weighted mean. For example, a surface weighted mean of 100 µm, can have the smallest particles of 3 µm and the largest particles of 750 µm. A volume weighted mean would in this case be 250 µm.

### Collagen protein structure

In one embodiment of the invention the hydrogel layer comprises a collagen protein structure as an ECM-mimicking structure.

In one preferred embodiment of the invention, the ratio of the collagen protein structure to the dried nanocellulose is between 0.5:1 and 1:1.

In a preferred embodiment of the invention the collagen protein structure is selected from the group consisting of types I, II and III collagens and mixtures thereof.

Soluble collagens of the types I, II and III collagen are prepared by limited enzymatic digestion of tissue enriched in such types of collagen and subsequent formation of a collagen-based solution (i.e. a soluble collagen dissolved in a suitable solvent, such as diluted hydrochloric acid, diluted acetic acid or the like).

Insoluble collagens can be obtained from the following typical sources: type I collagen: bovine, chicken and fish skin, bovine and chicken tendons and bovine and chicken bones including fetal tissues; type II collagen: bovine articular cartilage, nasal septum, sternal cartilage; and type III collagen: bovine and human aorta and skin.

In another embodiment of the invention the collagen protein structure as used for the polymer layer is a human recombinant collagen.

The term "human recombinant collagen" refers to collagen manufactured by culturing a non-human organism genetically modified to express at least one human gene encoding a collagen. The human recombinant collagen is suitably selected from the group consisting of collagen type I, type II, type III, type IV, type V, type VI, type VII, type VIII, type IX, type X, type XI, type XII, type XIII, type XIV, type XV, type XVI, type XVII, type XVIII, type XIX, type XX, type XXI, type XXII, type XXIII, type XXIV, type XXV, type XXVI, and type XXVII. The collagen can be collagen of one type free of any other type, or can be a mixture of different types of collagen. Suitably, the collagen comprises or consists essentially of collagens selected from the group consisting of type I collagen, type III collagen and mixtures thereof.

Human recombinant collagen may be provided by any suitable method known in the art. For example, the step of providing human recombinant collagen may comprise the following protocol described in U. S. Pat. No. 5, 962, 648, the entire content of which is incorporated herein by reference. Further recombinant processes are set forth in U.S. Pat. No. 5,593,859 and WO2004/078120, which are also incorporated herein by reference. Preferably, collagen is produced by culturing a cell which has been genetically modified to express at least one gene encoding the polypeptide comprising collagen and additional genes encoding the subunits of the post-translational modifying enzyme prolyl 4-hydroxylase, followed by the purification of the resultant collagen monomer therefrom. The recombinant collagen solution may be subsequently subjected to polymerization or cross-linking conditions to produce insoluble fibrous collagen.

Bovine collagen is a mixture of collagen type I (85%) and collagen type III (15%), a combination which is set by nature and cannot be varied. An advantage of recombinant collagen is that collagen type I and collagen type III are made independently of one another, and so any combination of type I and type III collagen can be made. The products according to the present invention may suitably comprise human collagen type I and human collagen type III in any ratio.

For example, the products may comprise human collagen type I and human collagen type III in a ratio by weight of 100:0, 80:20, 60:40, 50:50, 40:60, 20:80 or 0:100, or anywhere in-between. Preferably, the ratio by weight of human collagen type I:human collagen type III is greater than about 50:50, and preferably it is greater than about 70:30, for example about 80:20. Suitably, the type I human recombinant collagen makes up at least about 75% by weight of the total human recombinant collagens in the material.

### Collagen-like protein structure

In one embodiment of the invention the ECM-mimicking structure is a collagen-like protein structure. In the context of the present invention the "collagen-like protein structure" is defined as a recombinant collagen-like protein of bacterial origin which comprises at least one MMP cleavage site. Typically, it represents a modular collagen-like protein that is stable at mammalian body temperature either in its native state or after chemical cross-linking and aggregates to form fibrils.

In one preferred embodiment of the invention, the ratio of the collagen-like protein structure to the dried nanocellulose is between 0.5:1 and 1:1.

In a preferred embodiment the collagen-like protein structure of the invention is defined by the distinctive supercoiled triple-helix conformation, having a (Gly-Xaa-Yaa)ₘ amino acid sequence. In this configuration, Gly provides a glycine residue with Xaa and Yaa independently comprising any known imino or amino acid residue for each repeat unit. Unique properties of the collagen triple-helix motif include its molecular hydrodynamic properties, extensive hydration, ability to bind diverse ligands, and capacity to self-associate to form fibrils and other higher order structures. These distinctive features have been exploited by nature to fill a wide range of structural and functional niches.

In a more preferred embodiment, the collagen-like protein structure comprises the formula I:

(I) [(Gly-Xaa-Yaa)ₘ-(insert)ₙ]ₚ

where m is between 1 to 200 and (Gly-Xaa-Yaa)ₘ represents a triple helical domain with Xaa and Yaa being independently any natural and unnatural imino or amino acid for each repeat unit, wherein the insert is comprised of 1 to 50 of any imino or amino acid, with n being 0 or 1, and p being any number from 2 to 10 wherein the value of n is unique for each repeat and at least one insert is provided in the collagen-like protein which comprises at least one MMP cleavage site.

The overall content of Xaa and Yaa provides a proline rich structure where the total percentage of proline of all residues in the Xaa and Yaa positions is greater than 19%, but optimally, though not exclusively, between 19.5 and 40%. Alternatively, or in conjunction with the proline concentration, the triple helical motif may also contain a concentration of charged residues (e.g. Asp, Glu, Lys, Arg, His) of greater than 14% and optimally, though not exclusively, between 14-35%. Such domains should also aggregate, either naturally or synthetically, at a neutral pH or otherwise using one or a variation of such protocols discussed herein or otherwise known in the art.

The triple helical domains may be isolated from one or multiple pathogenic or non-pathogenic bacterial organisms. By way of example, the triple helical domains can include domains derived from *Streptococcus pyogenes, Clostridium perfringens, Methylobacterium sp.*4-46, *Solibacter usitatus* Ellin6076 or *Rhodopseudomonas palustris* tie-1, which exhibit the desired heat stability in either its native state or after stabilization by chemical cross-linking. Such sequences may include those identified in U.S. Patent No. 6,953,839 or WO2010/091251, the contents of which are incorporated herein by reference. Alternatively, each triple helical domain may include repeats, fragments, homologues or combinations of the foregoing peptide sequences.

In a preferred embodiment of the invention the triple helical domains of the collagen-like protein structure are independently selected from the group consisting of SEQ ID No. 1, 2, 44, 45 and 46.
CL triple helical protein from *Streptococcus pyogenes* (SEQ ID No. 1) 
GSPGLPGPRGEQGPTGPTGPAGPRGLQGLQGERGEQGPTGPAGPRGLQGERGEQGPTG
LAGKAGEAGAKGETGPAGPQGPRGEQGPQGLPGKDGEAGAQGPAGPMGPAGERGEKGEPGT
QGAKGDRGETGPVGPRGERGEAGPAGKDGERGPVGPAGKDGQNGQDGLPGKDGKDGQNGKD
GLPGKDGKDGQNGKDGLPGKDGKDGQDGKDGLPGKDGKDGLPGKDGKDGQPGKPGKY
Triple helical protein from *Clostridium perfringens* (SEQ ID No. 2): 
GPRGPRGPQGEQGPQGERGFTGPQGPVGPQGEQGPQGERGFTGPQGPIGLQGEQGPQGER
GFTGPQGPVGPQGEQGPQGERGFTGPQGPVGPQGEQGPQGERGFTGPQGPIGPQGEQGPQ
GERGFTGPQGPIGPQGNQGPIGPQGEQGPQGATGPQGPQGPVGPQGNQGPIGPQGPVGPQ
GPQGQPGVN
Triple helical protein from *Methylobacterium sp.* 4-46 (SEQ ID No. 44): 
GLPGPKGDPGPQGPAGPKGEPGPKGEPGPKGEPGPKGEPGPKGEPGPKGEPGPKGEPGPK
GEPGPRGEAGPQGALGPKGEAGSRGEPGPRGEPGPKGEAGLAGAPGPKGEAGPRGPQGER
GPPGAPGAA
Triple helical protein from *Solibacter Usitatus Ellin6076* (SEQ ID No. 45): 
GPAGPAGPQGPAGPAGAQGPAGPAGPQGPAGPQGSAGAQGPKGDTGAAGPAGEAGPKGET
GAAGPKGDTGAAGPAGPKGDTGAAGPAGPKGDTGAAGATGPKGEKGETGAAGPKGDKGET
GAAGPKGDKGETGAAGPKGEKGETGAVGPKGDKGETGAAGPKGDRGETGAVGPKGDKGET
GAVGPKGDKGETGAIGPKGDKGDKGDKGDAGVAGPQGIQGVKGDTGLQGPKGDAGPQGAP
GTPGGG
Triple helical protein from *Rhodopseudomonas palustris tie-1* (SEQ ID No. 46):

GRPGPQGPRGRPGEPGRPGPQGHPGRPGPEGPRGKQGPVGKPGPQGKAGPQGKPGIAGKP
GPDGKPGPIGPQGKAGPQGPRGEQGLRGEQGPRGEQGPQGPRGEQGPRGEPGPAGAL

Also important to the stabilizing structure of the collagen-like products of the instant invention is the proper formation of the triple helical structure. While some triple helical domains are able to be formed using the native mechanisms of the expression vehicle, proper folding of the recombinant bacterial collagen-like structure represented by formula I may also be assisted using globular or variable (V) domains. Such globular domains may be expressed directly or indirectly to the N-terminus and/or the C-terminus of the triple helical domain and can facilitate both post-translational folding and refolding following heat denaturation. One non-limiting example of an N-terminus domain is the entirety of or a portion of a variable (V) domain the Scl2 sequence found in *Streptococcus pyogenes,* which may be provided as the following peptide sequence:

EENEKVREQEKLIQQLSEKLVEINDLQTLNGDKESIQSLVDYLTRRGKLEEEWME
YLNS GIQRKLFV (SEQ ID No. 42).

A non-limiting example of a C-terminus variable domain or V domain may be isolated from the organism *Rhodopseudomonas palustris* tie-1, which may be provided as the following peptide sequence:

PSIEQVMPWLHLIFDAYEDYKAQRAREARELEERLAAEALEQAAREAAEREV
AAAIEAANAEAEIMLDDETHAEGGKKKKKRKHKD (SEQ ID No. 43)

Further stabilizing structures such as "coiled coil forming sequences", C-propeptides or "foldons" may include those identified in WO2010/091251, the contents of which are incorporated herein by reference.

### Peptide

In one embodiment of the invention, the ECM-mimicking structure is a peptide comprising a MMP cleavage site.

In one preferred embodiment of the invention, the ratio of the ECM-mimicking peptide to the dried nanocellulose is between 0.5:1 and 1:1.

According to the invention, the term "peptide comprising a MMP cleavage site" is defined as an oligo- or polypeptide with less than 100 amino acids, preferably with less than 90, 80, 70, 60 or 50 amino acids, and more preferably with less than 40 or 30 amino acids, whereby the peptide is not a fragment of a collagen protein structure according to the invention and furthermore not a collagen-like protein structure according to the invention or fragment thereof. As used in the present invention this peptide is also denominated as "MMP-cleavable peptide"

In a preferred embodiment of the invention, the MMP cleavage site is given by one of the following amino acid sequences as given in single letter code:
- GPQG↓AGQ (SEQ ID No. 3),
- GPQG↓IWGQ (SEQ ID No. 4),
- GNVG↓LAGA (SEQ ID No. 5),
- GPQG↓IA (SEQ ID No. 6),
- GPQG↓IL (SEQ ID No. 7),
- GPQG↓LA (SEQ ID No. 8),
- GPQG↓LL (SEQ ID No. 9),
- GPLG↓IA (SEQ ID No. 10),
- GPLG↓IL (SEQ ID No. 11),
- GPLG↓LA (SEQ ID No. 12),
- GPLG↓LL (SEQ ID No. 13),
- GPRG↓LQ (SEQ ID No. 14),
- GPTG↓LA (SEQ ID No. 15),
- GPQGIAGQRGVVGLP (SEQ ID No. 16)
- GPQGLLGAPGILGLP (SEQ ID No. 17)
- GEQGPQGLP (SEQ ID No. 18)
- GPQGLAGQRGIV (SEQ ID No. 19)
- GAQGPPGAPGPLGIAGITGARGLAGPPGMPGPRGS (SEQ ID No. 20)
- GPLGIAGITGAR (SEQ ID No. 21)
- GAQGPPGAPGPLGIAGITGARGLA (SEQ ID No. 22)
- GPSGAEGPPGPQGLAGQRGIVGLPGQRGERGFP (SEQ ID No. 23)
- GPQGLAGQRGIV (SEQ ID No. 24)
- GPSGAEGPPGPQGLAGQRGIVGLP (SEQ ID No. 25)

In a preferred embodiment of the invention the MMP-cleavable peptide or the collagen like protein structure comprises one or more of the following:
a. a cellulose binding domain (CBD)
b. a heparin binding dipeptide motif (BA)ₙ, wherein B is an amino acid with a positively charged side chain, A is alanine an n is an integer between 4 and 20;
c. a pH-labile chemical linker which is preferably to the C-terminally or N-terminally attached to said peptide;
d. an integrin binding site, being preferably an RGD motif or having an amino acid sequence of GFPGER (SEQ ID No. 26) or GEFYFDLRLK (SEQ ID No. 27);
e. a photo-cleavable linker which is preferably C-terminally or N-terminally attached to said peptide;
f. an IKVAV (SEQ ID No. 28) motif;
g. a fibronectin binding domain, having preferably the amino acid sequence of GLAGQRGIVGLPGQRGER (SEQ ID No. 29);
h. a Discoidin domain-containing receptor 2 (DDR2) domain, having preferably an amino acid sequence of GPRGQPGVMGFP (SEQ ID No. 30);
i. a non-collagen-natural break having a peptide sequence spaced between two glycine residues of the insert sequence of the collagen-like protein structure; having an amino acid sequence of SEQ ID No. 31 to 37;
j. a heparin binding domain;
k. an α1β-integrin binding domain, being preferably GLPGER, GFPGER or GFPGEN (SEQ ID Nos. 38-40)
l. an ECM retaining moiety comprising the amino acid sequence DHLSDNYTLDHDRAIH (SEQ ID No. 41).

In a preferred embodiment of the invention the MMP-cleavable peptide or the collagen-like protein structure are covalently or non-covalently bound to the nanocellulose, and hereby preferably by the use of a cellulose-binding domain (CBD).

CBDs adsorbs specifically and tightly on cellulose. Thus, they are a useful tool to address a bioactive peptide to the cellulose surface, in a specific and flexible way. By binding bifunctional CBD containing recombinant proteins, the nanocellulose can be provided with further functionalities such as binding domains for ECM proteins such as fibronectin or cell membrane proteins such as integrins.

Chimeric proteins with a CBD have been disclosed in several U.S. patents such as US. Pat Nos. 5,202,247 B1 or US 6,407,208 B1. Moreover, Andrade et al., Inc. J Biomed Mater Res 92A: 9-17, 2010, discloses a bifunctional protein with CBD fused to RGD which improves the affinity of fibroblasts for bacterial cellulose.

In a further embodiment of the invention, the MMP-cleavable peptide or the collagen-like protein structure further contains a repetitive dipeptide motif (BA)ₙ, wherein B is an amino acid with a positively charged side chain, A is alanine and n is a number from 4 to 20, indicating the number of individual repeating dipeptide modules (BA) within the dipeptide motif (BA)ₙ. The amino acid B is preferably arginine, with the one-letter code R, or lysine, indicated by the one-letter code K. Except for the L- and D-variants of arginine and lysine as natural amino acids, moreover generally all non-natural amino acids which are positively charged (basic) are suitable as amino acid B. The repetitive dipeptide motif represents a minimal binding motif for highly negative charged oligo- or polysaccharides such as heparin which enables the incorporation of heparin within the nanocellulose layer. Preferred repetitive dipeptide motif (BA)ₙ are disclosed in WO2014/040591, the contents of which are incorporated herein by reference.

According to another embodiment of the invention, the MMP-cleavable peptide or the collagen-like protein structure comprises a light-cleavable chemical linker, an enzymatically cleavable linker or a pH-sensitive chemical linker or a combination thereof. Due to this linkers the peptide or the collagen-like protein structure could be cleaved off the nanocellulose or alternatively certain functional domain could be cleaved from the nanocellulose binding peptide or the collagen-like protein structure in order to control the functionality of the peptide.

In another embodiment of the invention, the MMP-cleavable peptide or the collagen-like protein structure further comprises an oligonucleotide sequence as an enzymatically cleavable linker, which is a substrate for nucleases.

In an embodiment of the invention, the MMP-cleavable peptide or the collagen-like protein structure further comprises an integrin binding domain, having preferably the sequence cycloRGDyK.

The MMP-cleavable peptide or the collagen-like protein structure might contain insert sequences to improve the bendability or elasticity of the biomaterial or otherwise serve as a natural binding domain or biological cleavage sequence. Natural breaks or interruption sequences, for example, may include those of non-fibrillary human collagens, which are typically provided as 1 to 50 amino acids spaced between two glycine residues. While the instant invention is not so limited, examples of such sequences include those provided as follows (SEQ ID Nos. 31 to 37) as well as repeats, fragments, homologues or combinations thereof:
- GAAVMG (SEQ ID No. 31),
- GDSAVILG (SEQ ID No. 32),
- GDMVVSRVKG (SEQ ID No. 33),
- GRLVDTGPGAREKG (SEQ ID No. 34),
- GSVPNVDRLLETAGIKASALREIVETWDESSGSFLPVPERRRG (SEQ ID No. 35),
- GPGEFYFDLRLKGPG (SEQ ID No. 36),
- GQISEQKRPIDVEFQK (SEQ ID No. 37).

In an alternative embodiment of the invention particulate ECM, collagen protein structure, collagen-like protein structure and MMP-cleavable peptide are not bound to the nanocellulose but are separated within the nanocellulose layer.

### Further characteristics of the wound care product

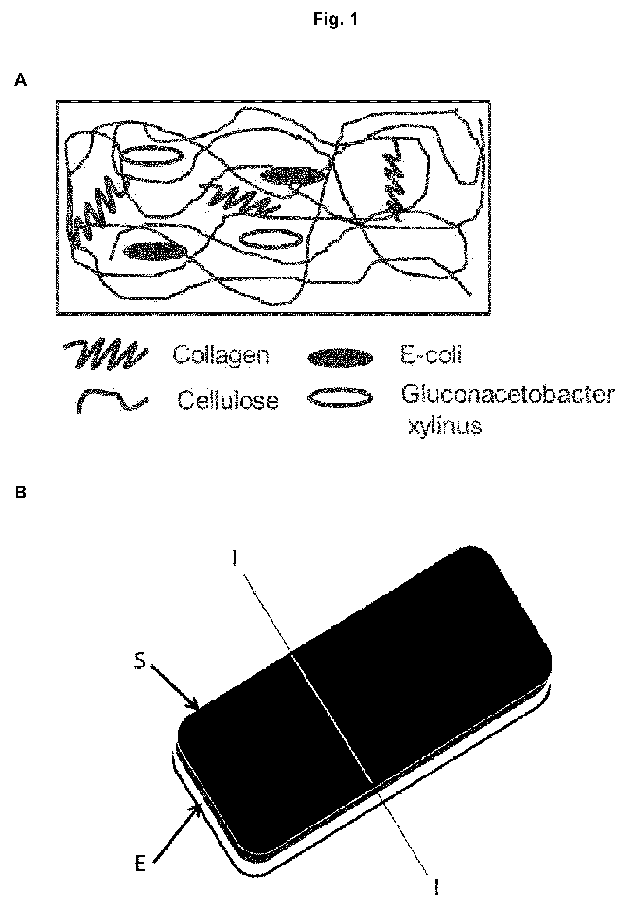

The dimension of the surface area of the wound care product may be selected according to the size of the wound. Typically, a wound care product of the present invention may have a surface area of from 5 cm² to 400cm², and particularly of from 25 cm² to 200cm², such as 100cm². Also the shape of the wound care product according to the present invention may vary with respect to the wound to be treated, and the present invention encompasses, for example, rectangular, square-, circle- or oval-shaped wound care products. For example, the wound care product may have a rectangular shape with rounded corners as depicted in Fig. 1B.

It shall be understood that the wound care product according to the present invention may include still further layers such as e.g. a covering layer.

In a preferred embodiment the covering layer is a polyurethane film having preferably a thickness between 10 and 50 µm, preferably between 15 and 40 µm and more preferably between 18 and 30 µm. The PU film is used to control water steam permeation and/or to control the fluid handling capacity. Thereby, this additional layer is useful to develop a wound care product that is useful for different exuding levels.

In a preferred embodiment of the invention the nanocellulose layer is hydrophobized or combined with a hydrophobic layer. Due to the hydrophobic properties the wound care product will adsorb bacteria which makes it especially suitable in split-skin harvesting or for burns, in particular when these wounds are already infected.

According to a first embodiment of the invention the wound dressing is characterized in that the bacteria-adsorbing design consists of the nanocellulose layer itself being designed hydrophobic. This hydrophobic design can be a treatment with dialkyl carbamoyl chloride and/or alkene ketene dimer as is known in the state of the art.

In preferred methods for the hydrophobic design of the nanocellulose layer such a layer has a hydrophobic design when its nanocellulose content is greater than approximately 5 wt.-%, preferably greater than 20 wt.-%, more preferably greater than 60 wt.-%, in particular preferably greater than 80 wt.-%, such as greater than 90 wt.-%, greater than 95 wt.-% or even greater than 97 wt.-%, such as for example 99 wt.-%.

In a second embodiment the bacteria-adsorbing design of the wound care product consists of the wound care product having, in addition to the nanocellulose layer, at least one hydrophobic layer on the side of the nanocellulose layer facing the wound.

This additional hydrophobic layer can contain cellulose acetate fabric, viscose fabric, cotton fabric or a mixed fabric, wherein hot-melt adhesive fibers are preferred as mixed fabric. Hydrophobic layers based on cellulose acetate fabric and cotton fabric, in particular cellulose acetate fabric, are particularly preferred. The hydrophobizing of the hydrophobic layer can consist of a treatment with dialkyl carbamoyl chloride and/or alkene ketene dimer. In a preferred embodiment the hydrophobic layer and the nanocellulose layer are grown together in one production process. Alternatively it is possible that there is an adhesive layer between the hydrophobic layer and the nanocellulose layer or that the two layers have been joined together by a lamination method.

If the base material used for producing the hydrophobic layer is naturally hydrophilic, as is the case for example with cellulose and viscose, this base material is treated with the help of a coating in order to produce the hydrophobic layer.

According to a third embodiment, in the case of the wound care product according to the invention the nanocellulose layer is designed hydrophobic and also a hydrophobic layer is present on the side of the nanocellulose layer facing the wound.

There can also be a cover layer on the side of the nanocellulose layer facing away from the wound. However, in a preferred embodiment there is no cover layer on the side of the nanocellulose layer facing away from the wound. Instead, the wound is bandaged normally.

In a preferred embodiment of the invention the wound care product has a moisture vapor transmission rate (MVTR) as measured according DIN ISO 13726-2 of more than 1000 g/m²/24h, preferably of more than 1500 g/m²/24h, and preferably of more than 2000 g/m²/24h. The parameters are a target for the test MVTR upright. And MVTR inverted is >20.000 g/m²/24h. For a wet product or hydrogel the DIN ISO 13726-2 should be adapted to measure only the vapor transmission rate and not a combination of dry loss and vapour transmission rate.

The following variants of the cellulose-nanofiber products are particularly preferred within the context of the present invention.

### 1. Very wet product (promotes rehydration of the wound)

The product is harvested in a static culture after 5-7 day of fermentation followed by depyrogenisation treatment, packing and sterilization. Sterilization is performed by gamma irradiation with 25 Gy using a Cobalt⁹⁰ source. The wet product contains the maximum water absorption capacity.

The product can donate water to the wound and is useful for dry wounds.

### 2. Hydrobalance product:

The product is harvested in a static culture after 5-7 day of fermentation. After depyrogenisation treatment, the product is subjected to a mechanical treatment such as pressing to remove partly the water. Finally the product is packed and sterilized. Sterilization is performed by gamma irradiation with 25 Gy using a Cobalt⁹⁰ source. The hydrobalance product contains still water to donate and absorbs fluid. The product can donate water to the wound and absorb wound fluid and therefore is useful for imbalanced wounds.

### 3. Dry product:

The product is harvested in a static culture after 5-7 day of fermentation. After depyrogenisation treatment, the product is subjected to a mechanical treatment such as pressing to remove most of the water and dried under ambient temperature to yield a dry sheet. Finally the product is packed and sterilized. Sterilization is performed by gamma irradiation with 25 Gy using a Cobalt⁹⁰ source. The dry product represents a membrane like material.

### 4. Freeze-dried product:

The product is harvested in a static culture after 5-7 day of fermentation. After depyrogenisation treatment, the product is freeze-dried. Finally the product is packed and sterilized. Sterilization is performed by gamma irradiation with 25 Gy using a Cobalt⁹⁰ source. The dry product is a sponge like material.

### 5. Cellulose nanofiber "paper"

By using *acetobacter xylinum* in a static culture or shaking culture with an appropriate culture medium, the incubation for 5 to 7 days leads to the generation of CNF. The CNF as prepared by this culture method is added to water and mixed for several minutes. The resulting pulp is given in a fine-mesh screen. After separating the liquid, the resulting cellulose layer is dried to result in a paper-like sheet.

The aforementioned CNF variants can be mixed with other fibers/sheet of fibers as for example with dialkyl carbamoyl chloride-coated fibers.

Furthermore, after dehydration of the CNF layer (e.g. by pressure) and subsequent soaking with a ECM-, collagen- or peptide containing liquid the functionalised peptides/proteins can be incorporated within the CNF layer in an easy and cost-efficient manner. Hereby the use of a purified and depyrogenated CNF is preferred.

### Use of the wound-care product

The wound care product of the invention is particularly useful in the treatment of acute wounds, burn wounds, chronic wounds, and/or surgical wounds. The wound care product of the present invention may further be used in plastic surgery as well as for tissue engineering.

As such it can be used for the treatment of burns, partial and full-thickness wounds, pressure ulcers, venous ulcers, arterial ulcers, diabetic ulcers, chronic vascular ulcers, draining wounds, tunneled or undermined wounds, surgical wounds such as donor sites/grafts, post-Mohs surgery, post-laser surgery, podiatric dehiscence, and wound dehiscence, trauma wounds such as abrasions, lacerations, first, second, or third degree burns, and skin tears.

In the field of tissue engineering the wound care product of the invention can be used for remodelling of soft tissue, bone, cartilage, ligaments and tendons or dental applications.

For the medical use, it is required that the wound care product of the invention is provided in sterile form. This can be achieved by packaging the sterile product in a bacterial tight material with a marking on the packing that the product is sterilized. Bacterial tight materials are well known to the person skilled in art.

In a second aspect the invention provides a method for preparation of a biocellulose scaffold with collagen-like protein structures contained therein comprising the following steps:
a. co-cultivation of bacteria recombinantly expressing and excreting biocellulose with bacteria recombinantly expressing and secreting a collagen-like protein structure; removal of the microorganisms;
b. Depyrogenisation;
c. Optionally physical modification such as pressing or partly dehydration;
d. packaging and sterilization.

In a third aspect the invention provides a method for preparation of a biocellulose scaffold with particulate ECM comprising the step of cultivating bacteria recombinantly expressing and excreting biocellulose in the presence of ECM particles.

In a fourth aspect the invention provides a method for preparation of a biocellulose scaffold with particulate ECM comprising the step of cultivating bacteria recombinantly coexpressing and excreting biocellulose and the MMP-cleavable peptide;

In a fifth aspect the invention provides a method for preparation of a biocellulose scaffold with particulate ECM comprising the step of cultivating bacteria recombinantly coexpressing and excreting biocellulose and the collagen like protein structure.

The fourth and the fifth aspect of the invention represent a preferred embodiment for the case that the biocellulose is combined with a further recombinantly expressed component such as the MMP-cleavable peptide or the collagen-like protein structure. By using a coexpression system with only one microorganism the preparation can be performed in an easy manner.

In a sixth aspect the invention provides a method for preparation of a biocellulose scaffold with particulate ECM comprising the followings step
a. cultivation of bacteria recombinantly expressing and excreting biocellulose
b. isolation of the CNF by alkaline treatment
c. adding the CNF as resulting from step b. in a culture medium comprising bacteria recombinantly expressing and releasing the ECM-mimicking peptides or proteins;
   isolation and purification of the CNF/peptide or CNF protein structure from the medium and the bacteria;
d. Optionally physical modification such as pressing or partly dehydration;
e. packaging and sterilization.

In a preferred embodiment of the invention the CNF as resulting from the bacterial culture is purified by incubation with sodium hydroxide, washed and added to a culture medium comprising bacteria recombinantly expressing and releasing the ECM-mimicking peptides/proteins. After several days of incubation, the CNF layer is removed from the culture, the bacteria are killed and removed and the functionalized CNF material is adjusted to the required water content (e.g. wet, partially dried, dried or freeze-dried).

### Brief description of the drawings:

These and other aspects of the invention will be apparent from and elucidated with reference to the embodiments described hereinafter.

The invention will now be described, by way of example, based on embodiments with reference to the accompanying drawings.
In the drawings:
Fig. 1 shows a principal sketch of the wound care product according to a first embodiment in cross section (**A**) or in a perspective view (**B**).

In the Figures, like numbers refer to like objects throughout. Objects in the Figures are not necessarily drawn to scale.

### Detailed description of embodiments:

Various embodiments of the invention will now be described by means of the Figures.

Fig. 1 shows a principal sketch of the wound care product according to a first embodiment in cross section (**A**) or in a perspective view (**B**). The wound care product is shown in a cross section/perspective view and may be applied as such to a skin wound. To this, the wound care product has a surface that will face the wound when being applied and will come in direct contact with the wound, and has an opposite surface that will face away from the wound and will thus initially not contact the wound when applied. Fig. 1A shows the ECM-functionalized nanocellulose layer in a rather early production stage, whereby the co-cultivated recombinant *E.coli* bacteria expressing collagen-like protein and the recombinant *Gluconacetobacter xylinus* expressing the nanocellulose together produce a mesh of nanocellulose fibers with embedded collagen proteins. In the embodiment depicted in Fig. 1B, the upper layer may be considered as the scaffold of nanocellulose and at least one ECM-mimicking structure (S) and the lower layer may be considered a hydrogel layer (E) that has been applied to the ECM-functionalized nanocellulose layer.

While the invention has been illustrated and described in detail in the drawings and the foregoing description, such illustration and description are to be considered illustrative or exemplary and not restrictive.

From reading the present disclosure, other modifications will be apparent to persons skilled in the art. Such modifications may involve other features which are already known in the art and which may be used instead of or in addition to features already described herein.

Variations to the disclosed embodiments can be understood and effected by those skilled in the art, from a study of the drawings, the disclosure and the appended claims. In the claims, the word "comprising" does not exclude other elements or steps, and the indefinite article "a" or "an" does not exclude a plurality of elements or steps. The mere fact that certain measures are recited in mutually different dependent claims does not indicate that a combination of these measures cannot be used to advantage.

Any reference signs in the claims should not be construed as limiting the scope thereof.

### Definitions

The term "decellularized ECM" as used herein refers to an ECM tissue from which a substantial amount of cellular and nucleic acid content has been removed leaving behind a complex interstitial structure of ECM that can be used as a scaffold for wound healing or tissue regeneration. In a preferred embodiment said term refers to an ECM material that has been cleaned, disinfected, sterilized and optionally cross-linked.

### List of reference signs:

- S: Scaffold layer of nanocellulose and at least one ECM-mimicking structure
- E: Hydrophobic layer facing the wound

## Claims

1. A wound care product comprising a scaffold of nanocellulose and at least one ECM-mimicking structure which is selected from the group consisting of particulate ECM, collagen protein structure, collagen-like protein structure and peptide comprising a matrix metalloproteinase (MMP) cleavage site, wherein the at least one ECM-mimicking structure is contained within said nanocellulose scaffold.

2. Wound care product according claim 1, wherein the nanocellulose layer comprises nanocellulose selected from the group consisting of cellulose nanofibers (CNF), nanocrystalline cellulose and biocellulose produced by bacteria (BNC).

3. Wound care product according claim 1 or 2, wherein the BNC is produced by a microorganism of the group consisting of *Acetobacter xylinum, Sphaerotilus spp., Pseudomonas spp., Alcaligenes spp., Salmonella spp., Agrobacterium spp., Rhizobium spp.* and cyanobacterium expressing the exogenous acsAB genes from the cellulose synthase operon, and combinations thereof.

4. Wound care product according claims 1 to 3, wherein the ECM particulate is derived by micronizing in vivo prepared ECM.

5. Wound care product according to any of the above claims, wherein the collagen protein structure is selected from Type I, Type II or type III collagen or mixtures thereof.

6. Wound care product according to any of the above claims, wherein the collagen-like protein structure comprises the formula:
[(Gly-Xaa-Yaa)ₘ-(insert)ₙ]ₚ
where m is between 1 to 200 and (Gly-Xaa-Yaa)ₘ represents a triple helical domain with Xaa and Yaa being independently any natural and unnatural imino or amino acid for each repeat unit, wherein the insert is comprised of 1 to 50 of any imino or amino acid, with n being 0 or 1, and p being any number from 2 to 10 wherein the value of n is unique for each repeat and at least one insert is provided in the collagen-like protein which comprises at least one MMP cleavage site.

7. Wound care product according to claim 6, wherein the collagen-like protein structure is a bacterial protein, which is preferably recombinantly expressed.

8. Wound care product according to claim 7, wherein the triple helical domains of the collagen-like protein structure are independently selected from the group consisting of SEQ ID No. 1, 2, 44, 45 and 46.

9. Wound care product according to claim 7 or 8, wherein the collagen-like protein structure further comprises as its C-terminus or N-terminus a non-collagenous domain which facilitates protein folding of the triple helical domains.

10. Wound care product according to claim 9, wherein the non-collagenous domain is selected from the group consisting of a foldon, a coiled coil sequence, and a C-propeptide.

11. Wound care product according to any of the above claims, wherein the peptide comprising the MMP cleavage and/or the collagen-like protein structure are covalently or non-covalently bound to the nanocellulose, and hereby preferably by a cellulose-binding domain (CBD).

12. Wound care product according to any of the above claims, wherein the MMP cleavage site of said MMP-cleavable peptide or of the collagen-like protein structure is selected from the group consisting of SEQ ID No. 6 to 30, fragments thereof and combinations thereof.

13. The wound care product according to any of the above claims, wherein the peptide comprising a MMP cleavage site or the collagen like protein structure comprises one or more of the following:
a. A cellulose binding domain (CBD);
b. a heparin binding dipeptide motif (BA)ₙ, wherein B is an amino acid with a positively charged side chain, A is alanine an n is an integer between 4 and 20;
c. a pH-labile chemical linker which is preferably attached to the C-terminus or N-terminus of said peptide;
d. an integrin binding site, being preferably an RGD motif having or an amino acid sequence of GFPGER (SEQ ID No. 26) or GEFYFDLRLK (SEQ ID NO. 27);
e. a photo-cleavable linker which is preferably C-terminally or N-terminally attached to said peptide;
f. an IKVAV (SEQ ID No. 28) motif;
g. a fibronectin binding domain, having preferably the amino acid sequence of GLAGQRGIVGLPGQRGER (SEQ ID No. 29);
h. a Discoidin domain-containing receptor 2 (DDR2) domain, having preferably an amino acid sequence of GPRGQPGVMGFP (SEQ ID No. 30);
i. a non-collagen-natural break having a peptide sequence spaced between two glycine residues of the insert sequence of the collagen-like protein structure; having an amino acid sequence of SEQ ID No. 31 to 37;
j. a heparin binding domain;
k. an α1β-integrin binding domain, being preferably GLPGER, GFPGER or GFPGEN (SEQ ID Nos. 38-40)
l. an ECM retaining moiety comprising the amino acid sequence DHLSDNYTLDHDRAIH (SEQ ID No. 41);
and whereby said peptide or collage-like protein structure is preferentially bound in a covalent or non-covalent form to the nanocellulose of the nanocellulose layer.

14. Wound care product according to any of the above claims, wherein the nanocellulose layer further comprises at least one cytokine, growth factor, hormone, or ECM-derived protein or any combination thereof.

15. The multi-layered wound care product according to any of the above claims for use in the treatment of acute and chronic wounds, comprising burns, partial and full-thickness wounds, pressure ulcers, venous ulcers, arterial ulcers, diabetic ulcers, chronic vascular ulcers, draining wounds, tunnelled or undermined wounds, surgical wounds such as donor sites/grafts, post-Mohs surgery, post-laser surgery, podiatric dehiscence, and wound dehiscence, trauma wounds such as abrasions, lacerations, first-, second- or third degree burns, and skin tears.

16. Method for preparation of a biocellulose scaffold with collagen-like protein structures contained therein comprising the following steps:
a. co-cultivation of bacteria recombinantly expressing and excreting biocellulose with bacteria recombinantly expressing and secreting a collagen-like protein structure;
b. isolation and purification of the composite structure of biocellulose and collagen-like protein from the medium and from the bacteria;
c. optionally depyrogenisation;
d. optionally physical modification such as pressing or partly dehydration;
e. packaging and sterilization.

17. Method for preparation of a biocellulose scaffold with particulate ECM comprising the following steps:
a. cultivation of bacteria recombinantly expressing and excreting biocellulose
b. isolation of the CNF by alkaline treatment
c. adding the CNF as resulting from step b. in a culture medium comprising bacteria recombinantly expressing and releasing the ECM-mimicking peptides or proteins;
d. isolation and purification of the CNF/peptide or CNF protein structure from the medium and the bacteria;
e. optionally depyrogenisation;
f. optionally physical modification such as pressing or partly dehydration
g. packaging and sterilization.


SEQUENCE LISTING

<110> BSN Medical GmbH

<120> Wound care product comprising ECM-functionalized nanocellulose

<130> 14719-PT-EP

<160> 46

<170> BiSSAP 1.3.5

<210> 1
<211> 240
<212> PRT
<213> Streptococcus pyogenes

<223> "Peptide for cross-linking hydrogel"

<220>
<223> Peptide for cross-linking hydrogel

<400> 1
Gly Ser Pro Gly Leu Pro Gly Pro Arg Gly Glu Gln Gly Pro Thr Gly
1               5                   10                  15
Pro Thr Gly Pro Ala Gly Pro Arg Gly Leu Gln Gly Leu Gln Gly Leu
            20                  25                  30
Gln Gly Glu Arg Gly Glu Gln Gly Pro Thr Gly Pro Ala Gly Pro Arg
        35                  40                  45
Gly Leu Gln Gly Glu Arg Gly Glu Gln Gly Pro Thr Gly Leu Ala Gly
    50                  55                  60
Lys Ala Gly Glu Ala Gly Ala Lys Gly Glu Thr Gly Pro Ala Gly Pro
65                  70                  75                  80
Gln Gly Pro Arg Gly Glu Gln Gly Pro Gln Gly Leu Pro Gly Lys Asp
                85                  90                  95
Gly Glu Ala Gly Ala Gln Gly Pro Ala Gly Pro Met Gly Pro Ala Gly
            100                 105                 110
Glu Arg Gly Glu Lys Gly Glu Pro Gly Thr Gln Gly Ala Lys Gly Asp
        115                 120                 125
Arg Gly Glu Thr Gly Pro Val Gly Pro Arg Gly Glu Arg Gly Glu Ala
    130                 135                 140
Gly Pro Ala Gly Lys Asp Gly Glu Arg Gly Pro Val Gly Pro Ala Gly
145                 150                 155                 160
Lys Asp Gly Gln Asn Gly Gln Asp Gly Leu Pro Gly Lys Asp Gly Lys
                165                 170                 175
Asp Gly Gln Asn Gly Lys Asp Gly Leu Pro Gly Lys Asp Gly Lys Asp
            180                 185                 190
Gly Gln Asn Gly Lys Asp Gly Leu Pro Gly Lys Asp Gly Lys Asp Gly
        195                 200                 205
Gln Asp Gly Lys Asp Gly Leu Pro Gly Lys Asp Gly Lys Asp Gly Leu
    210                 215                 220
Pro Gly Lys Asp Gly Lys Asp Gly Gln Pro Gly Lys Pro Gly Lys Tyr
225                 230                 235                 240

<210> 2
<211> 189
<212> PRT
<213> Clostridium perfringens

<223> "Peptide for cross-linking hydrogel"

<220>
<223> Peptide for cross-linking hydrogel

<400> 2
Gly Pro Arg Gly Pro Arg Gly Pro Gln Gly Glu Gln Gly Pro Gln Gly
1               5                   10                  15
Glu Arg Gly Phe Thr Gly Pro Gln Gly Pro Val Gly Pro Gln Gly Glu
            20                  25                  30
Gln Gly Pro Gln Gly Glu Arg Gly Phe Thr Gly Pro Gln Gly Pro Ile
        35                  40                  45
Gly Leu Gln Gly Glu Gln Gly Pro Gln Gly Glu Arg Gly Phe Thr Gly
    50                  55                  60
Pro Gln Gly Pro Val Gly Pro Gln Gly Glu Gln Gly Pro Gln Gly Glu
65                  70                  75                  80
Arg Gly Phe Thr Gly Pro Gln Gly Pro Val Gly Pro Gln Gly Glu Gln
                85                  90                  95
Gly Pro Gln Gly Glu Arg Gly Phe Thr Gly Pro Gln Gly Pro Ile Gly
            100                 105                 110
Pro Gln Gly Glu Gln Gly Pro Gln Gly Glu Arg Gly Phe Thr Gly Pro
        115                 120                 125
Gln Gly Pro Ile Gly Pro Gln Gly Asn Gln Gly Pro Ile Gly Pro Gln
    130                 135                 140
Gly Glu Gln Gly Pro Gln Gly Ala Thr Gly Pro Gln Gly Pro Gln Gly
145                 150                 155                 160
Pro Val Gly Pro Gln Gly Asn Gln Gly Pro Ile Gly Pro Gln Gly Pro
                165                 170                 175
Val Gly Pro Gln Gly Pro Gln Gly Gln Pro Gly Val Asn
            180                 185

<210> 3
<211> 8
<212> PRT
<213> Artificial Sequence

<223> "Cleavage after Gly4"

<220>
<223> Cleavage after Gly4

<400> 3
Gly Pro Gln Gly Ile Ala Gly Gln
1               5

<210> 4
<211> 8
<212> PRT
<213> Artificial Sequence

<223> "Cleavage after Gly4"

<220>
<223> Cleavage after Gly4

<400> 4
Gly Pro Gln Gly Ile Trp Gly Gln
1               5

<210> 5
<211> 8
<212> PRT
<213> Artificial Sequence

<223> "Cleavage after Gly4"

<220>
<223> Cleavage after Gly4

<400> 5
Gly Asn Val Gly Leu Ala Gly Ala
1               5

<210> 6
<211> 6
<212> PRT
<213> Artificial Sequence

<223> "Cleavage after Gly4"

<220>
<223> Cleavage after Gly4

<400> 6
Gly Pro Gln Gly Ile Ala
1               5

<210> 7
<211> 6
<212> PRT
<213> Artificial Sequence

<223> "Cleavage after Gly4"

<220>
<223> Cleavage after Gly4

<400> 7
Gly Pro Gln Gly Ile Leu
1               5

<210> 8
<211> 6
<212> PRT
<213> Artificial Sequence

<223> "Cleavage after Gly4"

<220>
<223> Cleavage after Gly4

<400> 8
Gly Pro Gln Gly Leu Ala
1               5

<210> 9
<211> 6
<212> PRT
<213> Artificial Sequence

<223> "Cleavage after Gly4"

<220>
<223> Cleavage after Gly4

<400> 9
Gly Pro Gln Gly Leu Leu
1               5

<210> 10
<211> 6
<212> PRT
<213> Artificial Sequence

<223> "Cleavage after Gly4"

<220>
<223> Cleavage after Gly4

<400> 10
Gly Pro Leu Gly Ile Ala
1               5

<210> 11
<211> 6
<212> PRT
<213> Artificial Sequence

<223> "Cleavage after Gly4"

<220>
<223> Cleavage after Gly4

<400> 11
Gly Pro Leu Gly Ile Leu
1               5

<210> 12
<211> 6
<212> PRT
<213> Artificial Sequence

<223> "Cleavage after Gly4"

<220>
<223> Cleavage after Gly4

<400> 12
Gly Pro Leu Gly Leu Ala
1               5

<210> 13
<211> 6
<212> PRT
<213> Artificial Sequence

<223> "Cleveage after Gly4"

<220>
<223> Cleveage after Gly4

<400> 13
Gly Pro Leu Gly Leu Leu
1               5

<210> 14
<211> 6
<212> PRT
<213> Artificial Sequence

<223> "Cleavage after Gly4"

<220>
<223> Cleavage after Gly4

<400> 14
Gly Pro Arg Gly Leu Gln
1               5

<210> 15
<211> 6
<212> PRT
<213> Artificial Sequence

<223> "Cleavage after Gly4"

<220>
<223> Cleavage after Gly4

<400> 15
Gly Pro Thr Gly Leu Ala
1               5

<210> 16
<211> 15
<212> PRT
<213> Homo sapiens

<223> "Collagen fragment"

<220>
<223> Collagen fragment

<400> 16
Gly Pro Gln Gly Ile Ala Gly Gln Arg Gly Val Val Gly Leu Pro
1               5                   10                  15

<210> 17
<211> 15
<212> PRT
<213> Homo sapiens

<223> "collagen fragment"

<220>
<223> collagen fragment

<400> 17
Gly Pro Gln Gly Leu Leu Gly Ala Pro Gly Ile Leu Gly Leu Pro
1               5                   10                  15

<210> 18
<211> 9
<212> PRT
<213> Homo sapiens

<223> "repeat tripeptide sequence"

<220>
<223> repeat tripeptide sequence

<400> 18
Gly Glu Gln Gly Pro Gln Gly Leu Pro
1               5

<210> 19
<211> 12
<212> PRT
<213> Homo sapiens

<223> "repeat tripeptide sequence"

<220>
<223> repeat tripeptide sequence

<400> 19
Gly Pro Gln Gly Leu Ala Gly Gln Arg Gly Ile Val
1               5                   10

<210> 20
<211> 35
<212> PRT
<213> Homo sapiens

<223> "repeat tripeptide sequence"

<220>
<223> repeat tripeptide sequence

<400> 20
Gly Ala Gln Gly Pro Pro Gly Ala Pro Gly Pro Leu Gly Ile Ala Gly
1               5                   10                  15
Ile Thr Gly Ala Arg Gly Leu Ala Gly Pro Pro Gly Met Pro Gly Pro
            20                  25                  30
Arg Gly Ser
        35

<210> 21
<211> 12
<212> PRT
<213> Homo sapiens

<223> "repeat tripeptide sequence"

<220>
<223> repeat tripeptide sequence

<400> 21
Gly Pro Leu Gly Ile Ala Gly Ile Thr Gly Ala Arg
1               5                   10

<210> 22
<211> 24
<212> PRT
<213> Homo sapiens


<400> 22
Gly Ala Gln Gly Pro Pro Gly Ala Pro Gly Pro Leu Gly Ile Ala Gly
1               5                   10                  15
Ile Thr Gly Ala Arg Gly Leu Ala
            20

<210> 23
<211> 33
<212> PRT
<213> Homo sapiens

<223> "repeat tripeptide sequence"

<220>
<223> repeat tripeptide sequence

<400> 23
Gly Pro Ser Gly Ala Glu Gly Pro Pro Gly Pro Gln Gly Leu Ala Gly
1               5                   10                  15
Gln Arg Gly Ile Val Gly Leu Pro Gly Gln Arg Gly Glu Arg Gly Phe
            20                  25                  30
Pro


<210> 24
<211> 12
<212> PRT
<213> Homo sapiens

<223> "repeat tripeptide sequence"

<220>
<223> repeat tripeptide sequence

<400> 24
Gly Pro Gln Gly Leu Ala Gly Gln Arg Gly Ile Val
1               5                   10

<210> 25
<211> 24
<212> PRT
<213> Homo sapiens

<223> "repeat tripeptide sequence"

<220>
<223> repeat tripeptide sequence

<400> 25
Gly Pro Ser Gly Ala Glu Gly Pro Pro Gly Pro Gln Gly Leu Ala Gly
1               5                   10                  15
Gln Arg Gly Ile Val Gly Leu Pro
            20

<210> 26
<211> 6
<212> PRT
<213> Homo sapiens

<223> "Ã\216Â±2Ã\216Â²1 integrin binding site"

<220>
<223> Ã\216Â±2Ã\216Â²1 integrin binding site

<400> 26
Gly Phe Pro Gly Glu Arg
1               5

<210> 27
<211> 10
<212> PRT
<213> Homo sapiens

<223> "Ã\216Â±2Ã\216Â²1 integrin binding site"

<220>
<223> Ã\216Â±2Ã\216Â²1 integrin binding site

<400> 27
Gly Glu Phe Tyr Phe Asp Leu Arg Leu Lys
1               5                   10

<210> 28
<211> 5
<212> PRT
<213> Mus musculus

<223> "laminin alpha1 chain peptide"

<220>
<223> laminin alpha1 chain peptide

<400> 28
Ile Lys Val Ala Val
1               5

<210> 29
<211> 18
<212> PRT
<213> Homo sapiens

<223> "collagen derived sequence"

<220>
<223> collagen derived sequence

<400> 29
Gly Leu Ala Gly Gln Arg Gly Ile Val Gly Leu Pro Gly Gln Arg Gly
1               5                   10                  15
Glu Arg

<210> 30
<211> 12
<212> PRT
<213> Homo sapiens

<223> "Discoidin domain-containing receptor 2 (DDR2) domain"

<220>
<223> Discoidin domain-containing receptor 2 (DDR2) domain

<400> 30
Gly Pro Arg Gly Gln Pro Gly Val Met Gly Phe Pro
1               5                   10

<210> 31
<211> 6
<212> PRT
<213> Homo sapiens

<223> " from Ã\216Â±5 chain of type IV collagen"

<220>
<223> from Ã\216Â±5 chain of type IV collagen

<400> 31
Gly Ala Ala Val Met Gly
1               5

<210> 32
<211> 8
<212> PRT
<213> Homo sapiens

<223> "from Ã\216Â±1 (VII) collagen"

<220>
<223> from Ã\216Â±1 (VII) collagen

<400> 32
Gly Asp Ser Ala Val Ile Leu Gly
1               5

<210> 33
<211> 10
<212> PRT
<213> Homo sapiens

<223> "from Ã\216Â±4 (IV) collagen"

<220>
<223> from Ã\216Â±4 (IV) collagen

<400> 33
Gly Asp Met Val Val Ser Arg Val Lys Gly
1               5                   10

<210> 34
<211> 14
<212> PRT
<213> Homo sapiens

<223> "from Ã\216Â±1 (VII) collagen"

<220>
<223> from Ã\216Â±1 (VII) collagen

<400> 34
Gly Arg Leu Val Asp Thr Gly Pro Gly Ala Arg Glu Lys Gly
1               5                   10

<210> 35
<211> 43
<212> PRT
<213> Homo sapiens

<223> "from Ã\216Â±1 (VII) collagen"

<220>
<223> from Ã\216Â±1 (VII) collagen

<400> 35
Gly Ser Val Pro Asn Val Asp Arg Leu Leu Glu Thr Ala Gly Ile Lys
1               5                   10                  15
Ala Ser Ala Leu Arg Glu Ile Val Glu Thr Trp Asp Glu Ser Ser Gly
            20                  25                  30
Ser Phe Leu Pro Val Pro Glu Arg Arg Arg Gly
        35                  40

<210> 36
<211> 15
<212> PRT
<213> Homo sapiens

<400> 36
Gly Pro Gly Glu Phe Tyr Phe Asp Leu Arg Leu Lys Gly Pro Gly
1               5                   10                  15

<210> 37
<211> 16
<212> PRT
<213> Homo sapiens

<400> 37
Gly Gln Ile Ser Glu Gln Lys Arg Pro Ile Asp Val Glu Phe Gln Lys
1               5                   10                  15

<210> 38
<211> 6
<212> PRT
<213> Homo sapiens

<400> 38
Gly Leu Pro Gly Glu Arg
1               5

<210> 39
<211> 6
<212> PRT
<213> Homo sapiens

<400> 39
Gly Phe Pro Gly Glu Arg
1               5

<210> 40
<211> 6
<212> PRT
<213> Homo sapiens

<400> 40
Gly Phe Pro Gly Glu Asn
1               5

<210> 41
<211> 16
<212> PRT
<213> Homo sapiens

<400> 41
Asp His Leu Ser Asp Asn Tyr Thr Leu Asp His Asp Arg Ala Ile His
1               5                   10                  15

<210> 42
<211> 67
<212> PRT
<213> Streptococcus pyogenes

<400> 42
Glu Glu Asn Glu Lys Val Arg Glu Gln Glu Lys Leu Ile Gln Gln Leu
1               5                   10                  15
Ser Glu Lys Leu Val Glu Ile Asn Asp Leu Gln Thr Leu Asn Gly Asp
            20                  25                  30
Lys Glu Ser Ile Gln Ser Leu Val Asp Tyr Leu Thr Arg Arg Gly Lys
        35                  40                  45
Leu Glu Glu Glu Trp Met Glu Tyr Leu Asn Ser Gly Ile Gln Arg Lys
    50                  55                  60
Leu Phe Val
65

<210> 43
<211> 86
<212> PRT
<213> Rhodopseudomonas palustris

<400> 43
Pro Ser Ile Glu Gln Val Met Pro Trp Leu His Leu Ile Phe Asp Ala
1               5                   10                  15
Tyr Glu Asp Tyr Lys Ala Gln Arg Ala Arg Glu Ala Arg Glu Leu Glu
            20                  25                  30
Glu Arg Leu Ala Ala Glu Ala Leu Glu Gln Ala Ala Arg Glu Ala Ala
        35                  40                  45
Glu Arg Glu Val Ala Ala Ala Ile Glu Ala Ala Asn Ala Glu Ala Glu
    50                  55                  60
Ile Met Leu Asp Asp Glu Thr His Ala Glu Gly Gly Lys Lys Lys Lys
65                  70                  75                  80
Lys Arg Lys His Lys Asp
                85

<210> 44
<211> 129
<212> PRT
<213> Methylobacterium sp.

<400> 44
Gly Leu Pro Gly Pro Lys Gly Asp Pro Gly Pro Gln Gly Pro Ala Gly
1               5                   10                  15
Pro Lys Gly Glu Pro Gly Pro Lys Gly Glu Pro Gly Pro Lys Gly Glu
            20                  25                  30
Pro Gly Pro Lys Gly Glu Pro Gly Pro Lys Gly Glu Pro Gly Pro Lys
        35                  40                  45
Gly Glu Pro Gly Pro Lys Gly Glu Pro Gly Pro Lys Gly Glu Pro Gly
    50                  55                  60
Pro Arg Gly Glu Ala Gly Pro Gln Gly Ala Leu Gly Pro Lys Gly Glu
65                  70                  75                  80
Ala Gly Ser Arg Gly Glu Pro Gly Pro Arg Gly Glu Pro Gly Pro Lys
                85                  90                  95
Gly Glu Ala Gly Leu Ala Gly Ala Pro Gly Pro Lys Gly Glu Ala Gly
            100                 105                 110
Pro Arg Gly Pro Gln Gly Glu Arg Gly Pro Pro Gly Ala Pro Gly Ala
        115                 120                 125

Ala

<210> 45
<211> 246
<212> PRT
<213> Solibacteres

<400> 45
Gly Pro Ala Gly Pro Ala Gly Pro Gln Gly Pro Ala Gly Pro Ala Gly
1               5                   10                  15
Ala Gln Gly Pro Ala Gly Pro Ala Gly Pro Gln Gly Pro Ala Gly Pro
            20                  25                  30
Gln Gly Ser Ala Gly Ala Gln Gly Pro Lys Gly Asp Thr Gly Ala Ala
        35                  40                  45
Gly Pro Ala Gly Glu Ala Gly Pro Lys Gly Glu Thr Gly Ala Ala Gly
    50                  55                  60
Pro Lys Gly Asp Thr Gly Ala Ala Gly Pro Ala Gly Pro Lys Gly Asp
65                  70                  75                  80
Thr Gly Ala Ala Gly Pro Ala Gly Pro Lys Gly Asp Thr Gly Ala Ala
                85                  90                  95
Gly Ala Thr Gly Pro Lys Gly Glu Lys Gly Glu Thr Gly Ala Ala Gly
            100                 105                 110
Pro Lys Gly Asp Lys Gly Glu Thr Gly Ala Ala Gly Pro Lys Gly Asp
        115                 120                 125
Lys Gly Glu Thr Gly Ala Ala Gly Pro Lys Gly Glu Lys Gly Glu Thr
    130                 135                 140
Gly Ala Val Gly Pro Lys Gly Asp Lys Gly Glu Thr Gly Ala Ala Gly
145                 150                 155                 160
Pro Lys Gly Asp Arg Gly Glu Thr Gly Ala Val Gly Pro Lys Gly Asp
                165                 170                 175
Lys Gly Glu Thr Gly Ala Val Gly Pro Lys Gly Asp Lys Gly Glu Thr
            180                 185                 190
Gly Ala Ile Gly Pro Lys Gly Asp Lys Gly Asp Lys Gly Asp Lys Gly
        195                 200                 205
Asp Ala Gly Val Ala Gly Pro Gln Gly Ile Gln Gly Val Lys Gly Asp
    210                 215                 220
Thr Gly Leu Gln Gly Pro Lys Gly Asp Ala Gly Pro Gln Gly Ala Pro
225                 230                 235                 240
Gly Thr Pro Gly Gly Gly
                245

<210> 46
<211> 117
<212> PRT
<213> Rhodopseudomonas palustris

<400> 46
Gly Arg Pro Gly Pro Gln Gly Pro Arg Gly Arg Pro Gly Glu Pro Gly
1               5                   10                  15
Arg Pro Gly Pro Gln Gly His Pro Gly Arg Pro Gly Pro Glu Gly Pro
            20                  25                  30
Arg Gly Lys Gln Gly Pro Val Gly Lys Pro Gly Pro Gln Gly Lys Ala
        35                  40                  45
Gly Pro Gln Gly Lys Pro Gly Ile Ala Gly Lys Pro Gly Pro Asp Gly
    50                  55                  60
Lys Pro Gly Pro Ile Gly Pro Gln Gly Lys Ala Gly Pro Gln Gly Pro
65                  70                  75                  80
Arg Gly Glu Gln Gly Leu Arg Gly Glu Gln Gly Pro Arg Gly Glu Gln
                85                  90                  95
Gly Pro Gln Gly Pro Arg Gly Glu Gln Gly Pro Arg Gly Glu Pro Gly

            100                 105                 110
Pro Ala Gly Ala Leu
        115